# EUROPEAN PATENT SPECIFICATION

(11) **EP 1 276 867 B1**
(45) Date of publication and mention of the grant of the patent: **23.08.2006**
(21) Application number: 01938027.8
(22) Date of filing: 21.03.2001
(51) Int. Cl.: C12N 15/12, C07K 14/705, C12Q 1/68, G01N 33/68, G01N 33/50, A61K 48/00, A61P 5/00, A61P 25/00

(54) **Use of human latrophilin-like G protein-coupled receptor in screening methods**
Verwendung des menschlichen Latrophilin-like G Protein-coupled Receptor in Screenings-Verfahren
Utilisation du récepteur humain couplé à la protéine G semblable à la latrophiline dans des procédures de criblage

(30) Priority: 22.03.2000 US 191103 P; 12.06.2000 US 210745 P
(43) Date of publication of application: 22.01.2003
(73) Proprietor: Bayer HealthCare AG, 51368 Leverkusen (DE)
(72) Inventor: RAMAKRISHNAN, Shyam, Brighton, MA 02135 (US)
(86) International application number: PCT/EP2001/003223
(87) International publication number: WO 2001/070969

(56) References cited:
- WO-A-01/75067
- DATABASE EM_EST [Online] (ID: HSM004191), 12 March 1999 (1999-03-12) "Homo sapiens mRNA; EST DKFZp434K0211_r1" Database accession no. AL039715 XP002198805
- SUGITA S ET AL: "ALPHA-LATROTOXIN RECEPTOR CIRL/LATROPHILIN 1 (CL1) DEFINES AN UNUSUAL FAMILY OF UBIQUITOUS G-PROTEIN-LINKED RECEPTORS" JOURNAL OF BIOLOGICAL CHEMISTRY, vol. 273, no. 49, 1998, pages 32715-32724, XP000946649 ISSN: 0021-9258 cited in the application
- DATABASE GSN [Online] 7 December 2001 (2001-12-07) Database accession no. AAL26439 XP002198806 -& WO 01 51628 A (MILLENNIUM PREDICTIVE MEDICINE) 19 July 2001 (2001-07-19)

### SEQUENZPROTOKOLL

<110> Bayer AG
<120> REGULATION OF HUMAN LATROPHILIN-LIKE G PROTEIN-COUPLED RECEPTOR
<130> Lio027 Foreign Countries
<140>
   <141>
<150> US 60/191,103
   <151> 2000-03-22
<150> US 60/210,745
   <151> 2000-06-12
<160> 2
<170> PatentIn Ver. 2.1
<210> 1
   <211> 479
   <212> DNA
   <213> Homo sapiens
<400> 1
   gcttcctcct tccctttcct caccaaagca aaaaagagaa ctcagaccaa ctgatgactc 60
   tctttacaag cttcaactca ttgcattccg caatggaaag ctttttccaa gccactggaa 120
   attcaacaaa tttggctgat gatggaaaac gacgtactgt ggttacccct gtgattctca 180
   ccaaaataga tggtgtgaat gtagataccc accacatccc tgttaatgtg acactgcgtc 240
   gaattgcaca tggagcagat gctgttgcag cccggtggga tttcgatttg ctgaacggac 300
   aaggaggctg gaagtcagat gggtgccata tactctattc agatgaaaat atcactacga 360
   ttcagtgcta ctcccttagt aactatgcag ttttaatgga tttgacggga tctgaactat 420
   acacccaggc ggncagcctc ctgcatcctg tggtttatac taccgccatc attctcctc  479
<210> 2
   <211> 159
   <212> PRT
   <213> Homo sapiens
<400> 2
   Phe Leu Leu Pro Phe Ser His Gln Ser Lys Lys Glu Asn Ser Asp Gln
   1               5                   10                  15
   Leu Met Thr Leu Phe Thr Ser Phe Asn Ser Leu His Ser Ala Met Glu
                   20                  25                  30
   Ser Phe Phe Gln Ala Thr Gly Asn Ser Thr Asn Leu Ala Asp Asp Gly
           35                  40                  45
   Lys Arg Arg Thr Val Val Thr Pro Val Ile Leu Thr Lys Ile Asp Gly
       50                  55                  60
   Val Asn Val Asp Thr His His Ile Pro Val Asn Val Thr Leu Arg Arg
   65                  70                  75                  80
   Ile Ala His Gly Ala Asp Ala Val Ala Ala Arg Trp Asp Phe Asp Leu
                   85                  90                  95
   Leu Asn Gly Gln Gly Gly Trp Lys Ser Asp Gly Cys His Ile Leu Tyr
               100                 105                 110
   Ser Asp Glu Asn Ile Thr Thr Ile Gln Cys Tyr Ser Leu Ser Asn Tyr
           115                 120                 125
   Ala Val Leu Met Asp Leu Thr Gly Ser Glu Leu Tyr Thr Gln Ala Xaa
       130                 135                 140
   Ser Leu Leu His Pro Val Val Tyr Thr Thr Ala Ile Ile Leu Leu
   145                 150                 155

## Description

### TECHNICAL FIELD OF THE INVENTION

The invention relates to the area of G-protein coupled receptors. More particularly, it relates to the area of human latrophilin-like G protein-coupled receptors and their use in drug screening.

### BACKGROUND OF THE INVENTION

### G-Protein Coupled Receptors

Many medically significant biological processes are mediated by signal transduction pathways that involve G-proteins (Lefkowitz, *Nature 351,* 353-354, 1991). The family of G-protein coupled receptors (GPCR) includes receptors for hormones, neurotransmitters, growth factors, and viruses. Specific examples of GPCRs include receptors for such diverse agents as dopamine, calcitonin, adrenergic hormones, endothelin, cAMP, adenosine, acetylcholine, serotonin, histamine, thrombin, kinin, follicle stimulating hormone, opsins, endothelial differentiation gene-1, rhodopsins, odorants, cytomegalovirus, G-proteins themselves, effector proteins such as phospholipase C, adenyl cyclase, and phosphodiesterase, and actuator proteins such as protein kinase A and protein kinase C.

GPCRs possess seven conserved membrane-spanning domains connecting at least eight divergent hydrophilic loops. GPCRs (also known as 7TM receptors) have been characterized as including these seven conserved hydrophobic stretches of about 20 to 30 amino acids, connecting at least eight divergent hydrophilic loops. Most GPCRs have single conserved cysteine residues in each of the first two extracellular loops, which form disulfide bonds that are believed to stabilize functional protein structure. The seven transmembrane regions are designated as TM1, TM2, TM3, TM4, TM5, TM6, and TM7. TM3 has been implicated in signal transduction.

Phosphorylation and lipidation (palmitylation or farnesylation) of cysteine residues can influence signal transduction of some GPCRs. Most GPCRs contain potential phosphorylation sites within the third cytoplasmic loop and/or the carboxy terminus. For several GPCRs, such as the β-adrenergic receptor, phosphorylation by protein kinase A and/or specific receptor kinases mediates receptor desensitization.

For some receptors, the ligand binding sites of GPCRs are believed to comprise hydrophilic sockets formed by several GPCR transmembrane domains. The hydrophilic sockets are surrounded by hydrophobic residues of the GPCRs. The hydrophilic side of each GPCR transmembrane helix is postulated to face inward and form a polar ligand binding site. TM3 has been implicated in several GPCRs as having a ligand binding site, such as the TM3 aspartate residue. TM5 serines, a TM6 asparagine, and TM6 or TM7 phenylalanines or tyrosines also are implicated in ligand binding.

GPCRs are coupled inside the cell by heterotrimeric G-proteins to various intracellular enzymes, ion channels, and transporters (*see* Johnson *et al*., *Endoc. Rev.* 10, 317-331, 1989). Different G-protein alpha-subunits preferentially stimulate particular effectors to modulate various biological functions in a cell. Phosphorylation of cytoplasmic residues of GPCRs is an important mechanism for the regulation of some GPCRs. For example, in one form of signal transduction, the effect of hormone binding is the activation inside the cell of the enzyme, adenylate cyclase. Enzyme activation by hormones is dependent on the presence of the nucleotide GTP. GTP also influences hormone binding. A G-protein connects the hormone receptor to adenylate cyclase. G-protein exchanges GTP for bound GDP when activated by a hormone receptor. The GTP-carrying form then binds to activated adenylate cyclase. Hydrolysis of GTP to GDP, catalyzed by the G-protein itself, returns the G-protein to its basal, inactive form. Thus, the G-protein serves a dual role, as an intermediate that relays the signal from receptor to effector, and as a clock that controls the duration of the signal.

Over the past 15 years, nearly 350 therapeutic agents targeting GPCRs receptors have been successfully introduced onto the market. This indicates that these receptors have an established, proven history as therapeutic targets. Clearly, there is an ongoing need for identification and characterization of further GPCRs which can play a role in preventing, ameliorating, or correcting dysfunctions or diseases including, but not limited to, infections such as bacterial, fungal, protozoan, and viral infections, particularly those caused by HIV viruses, pain, cancers, anorexia, bulimia, asthma, Parkinson's diseases, acute heart failure, hypotension, hypertension, urinary retention, osteoporosis, angina pectoris, myocardial infarction, ulcers, asthma, allergies, benign prostatic hypertrophy, and psychotic and neurological disorders, including anxiety, schizophrenia, manic depression, delirium, dementia, several mental retardation, and dyskinesias, such as Huntington's disease and Tourett's syndrome.

### Latrophilin (Alpha-Latrotoxin Receptor)

Alpha-latrotoxin (ALX), a neurotoxic protein contained in black widow spider venom, stimulates a robust release of neurotransmitters and the subsequent degeneration of the affected nerve terminals. This effect is mediated by ALX binding to a G protein-coupled receptor called latrophilin (Ichtchenko *et al., J. Biol. Chem. 274,* 5491-98, 1999; Rahman *et al., Philos. Trans. R. Soc. Lond B Biol. Sci. 28,* 39-86, 1999). Because of the dramatic effect of ALX on exocytosis, there is a need in the art to identify additional members of the latrophilin receptor family whose activity can be regulated to provide therapeutic effects.

The EST sequence identified by accession no. ALO39715 [XP002198805](DATABASE EM_EST[Online], [ID: HSM004191) was cloned from adult testis and discloses the sequence described by SEQ ID NO.:1.

### SUMMARY OF THE INVENTION

It is an object of the invention to provide reagents and methods of regulating a human latrophilin-like G protein-coupled receptor (LT-GPCR). This and other objects of the invention are provided by one or more of the embodiments described below.

Disclosed is a LT-GPCR polypeptide comprising the amino acid sequence shown in SEQ ID NO: 2.

An embodiment of the invention is a method of screening for agents which decrease the activity of LT-GPCR. A test compound is contacted with a LT-GPCR polypeptide comprising the amino acid sequence shown in SEQ ID NO: 2.

Binding between the test compound and the LT-GPCR polypeptide is detected. A test compound which binds to the LT-GPCR polypeptide is thereby identified as a potential agent for decreasing the activity of LT-GPCR.

Another embodiment of the invention is a method of screening for agents which decrease the activity of LT-GPCR. A test compound is contacted with a polynucleotide encoding a LT-GPCR polypeptide, wherein the polynucleotide comprises a nucleotide sequence selected from the group consisting of:
the nucleotide sequence shown in SEQ ID NO:1, or a polynucleotide the sequence of which deviates from the sequence defined by SEQ ID NO.:1 due to the degeneration of the genetic code. Binding of the test compound to the polynucleotide is detected. A test compound which binds to the polynucleotide is identified as a potential agent for decreasing the activity of LT-GPCR. The agent can work by decreasing the amount of the LT-GPCR through interacting with the LT-GPCR mRNA.

Another embodiment of the invention is a method of screening for agents which regulate the activity of LT-GPCR. A test compound is contacted with a LT-GPCR polypeptide comprising the amino acid sequence shown in SEQ ID NO: 2.

A LT-GPCR activity of the polypeptide is detected. A test compound which increases LT-GPCR activity of the polypeptide relative to LT-GPCR activity in the absence of the test compound is thereby identified as a potential agent for increasing the activity of LT-GPCR. A test compound which decreases LT-GPCR activity of the polypeptide relative to LT-GPCR activity in the absence of the test compound is thereby identified as a potential agent for decreasing the activity of LT-GPCR.

Even another embodiment of the invention is a method of screening for agents which decrease the activity of LT-GPCR. A test compound is contacted with a LT-GPCR product of a polynucleotide which comprises a nucleotide sequence selected from the group consisting of:
the nucleotide sequence shown in SEQ ID NO: 1, or a polynucleotide the sequence of which deviates from the sequence defined by SEQ ID NO.:1 due to the degeneration of the genetic code.

Binding of the test compound to the LT-GPCR product is detected. A test compound which binds to the LT-GPCR product is thereby identified as a potential agent for decreasing the activity of LT-GPCR.

Still another embodiment of the invention is a method of reducing the activity of LT-GPCR. A cell is contacted with a reagent which specifically binds to a polynucleotide encoding a LT-GPCR polypeptide or the product encoded by the polynucleotide, wherein the polynucleotide comprises a nucleotide sequence selected from the group consisting of:
the nucleotide sequence shown in SEQ ID NO: 1, or a polynucleotide the sequence of which deviates from the sequence defined by SEQ ID NO.:1 due to the degeneration of the genetic code. LT-GPCR activity in the cell is thereby decreased.

The invention thus provides a human latrophilin-like G protein-coupled receptor which can be used to identify test compounds which may act as agonists or antagonists at the receptor site. Human latrophilin-like G protein-coupled receptor and fragments thereof also are useful in raising specific antibodies which can block the receptor and effectively prevent ligand binding.

### BRIEF DESCRIPTION OF THE DRAWINGS

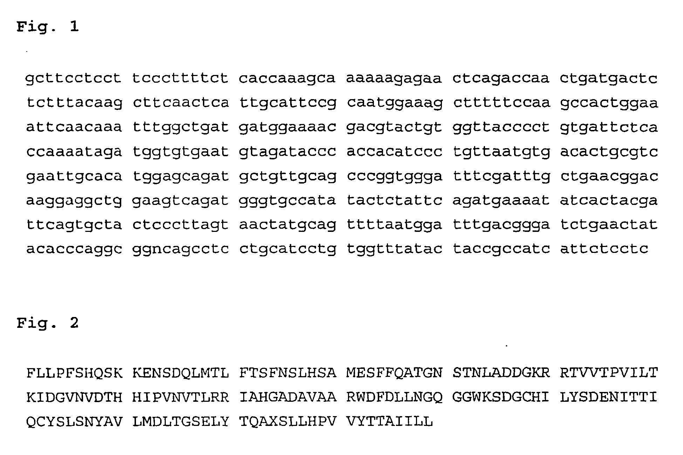

Fig. 1 shows the DNA-sequence encoding a LT-GPCR polypeptide.
Fig. 2 shows the amino acid sequence deduced from the DNA-sequence of Fig.1.

### DETAILED DESCRIPTION OF THE INVENTION

The invention relates to the use of an isolated polynucleotide encoding a LT-GPCR polypeptide and being selected from the group consisting of:
a) a polynucleotide encoding a LT-GPCR polypeptide comprising the amino acid sequence shown in SEQ ID NO: 2;
b) a polynucleotide comprising the sequence of SEQ ID NO: 1;
c) a polynucleotide the sequence of which deviates from the polynucleotide sequences specified in (a) to (b) due to the degeneration of the genetic code in drug screening.

Furthermore, it has been discovered by the present applicant, that a latrophilin-like G protein-coupled receptor (LT-GPCR) protein, particularly human LT-GPCR protein, can be used in therapeutic methods to treat disorders which involve neural or endocrine dysfunctions resulting from aberrant exocytosis. Human LT-GPCR also can be used to screen for LT-GPCR agonists and antagonists.

### LT-GPCR Polypeptides

LT-GPCR polypeptides according to the invention comprise an amino acid sequence shown in SEQ ID NO:2, a portion of one of those sequences, or a biologically active variant thereof, as defined below. An LT-GPCR polypeptide of the invention therefore can be a portion of an LT-GPCR protein, a full-length LT-GPCR protein, or a fusion protein comprising all or a portion of an LT-GPCR protein. A coding sequence for the amino acid sequence shown in SEQ ID NO:2 is shown in SEQ ID NO:1.

### Biologically Active Variants

LT-GPCR polypeptide variants which are biologically active, *i.e.,* retain the ability to bind a ligand to produce a biological effect, such as cyclic AMP formation, mobilization of intracellular calcium, or phosphoinositide metabolism, also are LT-GPCR polypeptides. Percent identity between a putative LT-GPCR polypeptide variant and an amino acid sequence of SEQ ID NO:2 is determined using the Blast2 alignment program.

Variations in percent identity can be due, for example, to amino acid substitutions, insertions, or deletions. Amino acid substitutions are defined as one for one amino acid replacements. They are conservative in nature when the substituted amino acid has similar structural and/or chemical properties. Examples of conservative replacements are substitution of a leucine with an isoleucine or valine, an aspartate with a glutamate, or a threonine with a serine.

Amino acid insertions or deletions are changes to or within an amino acid sequence. They typically fall in the range of about 1 to 5 amino acids. Guidance in determining which amino acid residues can be substituted, inserted, or deleted without abolishing biological or immunological activity of an LT-GPCR polypeptide can be found using computer programs well known in the art, such as DNASTAR software. Whether an amino acid change results in a biologically active LT-GPCR polypeptide can readily be determined by assaying for binding to a ligand or by conducting a functional assay, as described for example, in the specific Examples, below.

### Fusion Proteins

Fusion proteins can comprise at least 5, 6, 8, 10, 25, or 50 or more contiguous amino acids of an amino acid sequence shown in SEQ ID NO:2. Fusion proteins are useful for generating antibodies against LT-GPCR polypeptide amino acid sequences and for use in various assay systems. For example, fusion proteins can be used to identify proteins which interact with portions of an LT-GPCR polypeptide. Protein affinity chromatography or library-based assays for protein-protein interactions, such as the yeast two-hybrid or phage display systems, can be used for this purpose. Such methods are well known in the art and also can be used as drug screens.

An LT-GPCR polypeptide fusion protein comprises two polypeptide segments fused together by means of a peptide bond. The first polypeptide segment comprises at least 5, 6, 8, 10, 25, or 50 or more contiguous amino acids of SEQ ID NO:2 or from a biologically active variant, such as those described above. The first polypeptide segment also can comprise full-length LT-GPCR protein.

The second polypeptide segment can be a full-length protein or a protein fragment. Proteins commonly used in fusion protein construction include β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located between the LT-GPCR polypeptide-encoding sequence and the heterologous protein sequence, so that the LT-GPCR polypeptide can be cleaved and purified away from the heterologous moiety.

A fusion protein can be synthesized chemically, as is known in the art. Preferably, a fusion protein is produced by covalently linking two polypeptide segments or by standard procedures in the art of molecular biology. Recombinant DNA methods can be used to prepare fusion proteins, for example, by making a DNA construct which comprises coding sequences selected from SEQ ID NO:1 in proper reading frame with nucleotides encoding the second polypeptide segment and expressing the DNA construct in a host cell, as is known in the art. Many kits for constructing fusion proteins are available from companies such as Promega. Corporation (Madison, WI), Stratagene (La Jolla, CA), CLONTECH (Mountain View, CA), Santa Cruz Biotechnology (Santa Cruz, CA), MBL International Corporation (MIC; Watertown, MA), and Quantum Biotechnologies (Montreal, Canada; 1-888-DNA KITS).

### Identification of Species Homologs

Species homologs of human LT-GPCR polypeptide can be obtained using LT-GPCR polypeptide polynucleotides (described below) to make suitable probes or primers for screening cDNA expression libraries from other species, such as mice, monkeys, or yeast, identifying cDNAs which encode homologs of LT-GPCR polypeptide, and expressing the cDNAs as is known in the art.

### LT-GPCR Polynucleotides

An LT-GPCR polynucleotide can be single- or double-stranded and comprises a coding sequence or the complement of a coding sequence for an LT-GPCR polypeptide. A coding sequence for the human LT-GPCR polypeptide shown in SEQ ID NO:2 is shown in SEQ ID NO: 1. Percent sequence identity between the sequences of two polynucleotides is determined using computer programs such as ALIGN which employ the FASTA algorithm, using an affine gap search with a gap open penalty of -12 and a gap extension penalty of -2. Complementary DNA (cDNA) molecules, species homologs, and variants of LT-GPCR polynucleotides which encode biologically active LT-GPCR polypeptides also are LT-GPCR polynucleotides.

### Identification of Variants and Homologs of LT-GPCR Polynucleotides

Variants and homologs of the LT-GPCR polynucleotides described above also are LT-GPCR polynucleotides. Typically, homologous LT-GPCR polynucleotide sequences can be identified by hybridization of candidate polynucleotides to known LT-GPCR polynucleotides under stringent conditions, as is known in the art. For example, using the following wash conditions-2X SSC (0.3 M NaCl, 0.03 M sodium citrate, pH 7.0), 0.1% SDS, room temperature twice, 30 minutes each; then 2X SSC, 0.1% SDS, 50 °C once, 30 minutes; then 2X SSC, room temperature twice, 10 minutes each-homologous sequences can be identified which contain at most about 25-30% basepair mismatches. More preferably, homologous nucleic acid strands contain 15-25% basepair mismatches, even more preferably 5-15% basepair mismatches.

Species homologs of the LT-GPCR polynucleotides disclosed herein also can be identified by making suitable probes or primers and screening cDNA expression libraries from other species, such as mice, monkeys, or yeast. Human variants of LT-GPCR polynucleotides can be identified, for example, by screening human cDNA expression libraries. It is well known that the Tₘ of a double-stranded DNA decreases by 1-1.5 °C with every 1% decrease in homology (Bonner *et al., J. Mol. Biol. 81,* 123 (1973). Variants of human LT-GPCR polynucleotides or LT-GPCR polynucleotides of other species can therefore be identified by hybridizing a putative homologous LT-GPCR polynucleotide with a polynucleotide having a nucleotide sequence of SEQ ID NO: 1 or the complement thereof to form a test hybrid. The melting temperature of the test hybrid is compared with the melting temperature of a hybrid comprising LT-GPCR polynucleotides having perfectly complementary nucleotide sequences, and the number or percent of basepair mismatches within the test hybrid is calculated.

Nucleotide sequences which hybridize to LT-GPCR polynucleotides or their complements following stringent hybridization and/or wash conditions also are LT-GPCR polynucleotides. Stringent wash conditions are well known and understood in the art and are disclosed, for example, in Sambrook *et al*., MOLECULAR CLONING: A LABORATORY MANUAL, 2d ed., 1989, at pages 9.50-9.51.

Typically, for stringent hybridization conditions a combination of temperature and salt concentration should be chosen that is approximately 12-20 °C below the calculated Tₘ of the hybrid under study. The Tₘ of a hybrid between an LT-GPCR polynucleotide having a nucleotide sequence shown in SEQ ID NO:1 or the complement thereof and a polynucleotide sequence which is at least about 50, preferably about 75, 90, 96, or 98% identical to one of those nucleotide sequences can be calculated, for example, using the equation of Bolton and McCarthy, *Proc*. *Natl. Acad. Sci. USA. 48*, 1390 (1962):
Tₘ = 81.5 °C - 16.6(log₁₀[Na⁺]) + 0.41(%G + C) - 0.63(%formamide) - 600/*l*), where *l* = the length of the hybrid in basepairs.
Stringent wash conditions include, for example, 4X SSC at 65 °C, or 50% formamide, 4X SSC at 42 °C, or 0.5X SSC, 0.1% SDS at 65 °C. Highly stringent wash conditions include, for example, 0.2X SSC at 65 °C.

### Preparation of LT-GPCR Polynucleotides

A naturally occurring LT-GPCR polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated LT-GPCR polynucleotides. For example, restriction enzymes and probes can be used to isolate polynucleotide fragments which comprises LT-GPCR nucleotide sequences. Isolated polynucleotides are in preparations which are free or at least 70, 80, or 90% free of other molecules.

LT-GPCR cDNA molecules can be made with standard molecular biology techniques, using LT-GPCR mRNA as a template. LT-GPCR cDNA molecules can thereafter be replicated using molecular biology techniques known in the art and disclosed in manuals such as Sambrook *et al*. (1989). An amplification technique, such as PCR, can be used to obtain additional copies of polynucleotides of the invention, using either human genomic DNA or cDNA as a template.

Alternatively, synthetic chemistry techniques can be used to synthesizes LT-GPCR polynucleotides. The degeneracy of the genetic code allows alternate nucleotide sequences to be synthesized which will encode an LT-GPCR polypeptide having, for example, an amino acid sequence shown in SEQ ID NO:2 or a biologically active variant thereof.

### Extending LT-GPCR Polynucleotides

The nucleotide sequence shown in SEQ ID NO: 1 or its complement can be used to identify the corresponding full length gene from which it was derived. For example, contiguous nucleotide sequence selected from the complement of SEQ ID NO: 1 can be nick-translated or end-labeled with ³²P using polynucleotide kinase using labeling methods known to those with skill in the art (BASIC METHODS IN MOLECULAR BIOLOGY, Davis *et al*., eds., Elsevier Press, N.Y., 1986). For example, a lambda library prepared from human tissue can be screened directly with the labeled sequences of interest or the library can be converted en masse to pBluescript (Stratagene Cloning Systems, La Jolla, Calif. 92037) to facilitate bacterial colony screening (see Sambrook *et al*., 1989, pg. 1.20).

Both methods are well known in the art. Briefly, filters with bacterial colonies containing the library in pBluescript or bacterial lawns containing lambda plaques are denatured, and the DNA is fixed to the filters. The filters are hybridized with the labeled probe using hybridization conditions described by Davis *et al*., 1986. The partial sequences, cloned into lambda or pBluescript, can be used as positive controls to assess background binding and to adjust the hybridization and washing stringencies necessary for accurate clone identification. The resulting autoradiograms are compared to duplicate plates of colonies or plaques; each exposed spot corresponds to a positive colony or plaque. The colonies or plaques are selected and expanded, and the DNA is isolated from the colonies for further analysis and sequencing.

Positive cDNA clones are analyzed to determine the amount of additional sequence they contain using PCR with one primer from the partial sequence and the other primer from the vector. Clones with a larger vector-insert PCR product than the original partial sequence are analyzed by restriction digestion and DNA sequencing to determine whether they contain an insert of the same size or similar as the mRNA size determined from Northern blot analysis.

Once one or more overlapping cDNA clones are identified, the complete sequence of the clones can be determined, for example after exonuclease III digestion (McCombie *et al*., *Methods 3,* 33-40, 1991). A series of deletion clones are generated, each of which is sequenced. The resulting overlapping sequences are assembled into a single contiguous sequence of high redundancy (usually three to five overlapping sequences at each nucleotide position), resulting in a highly accurate final sequence.

Various PCR-based methods can be used to extend the nucleic acid sequences encoding the disclosed portions of human LT-GPCR to detect upstream sequences such as promoters and regulatory elements. For example, restriction-site PCR uses universal primers to retrieve unknown sequence adjacent to a known locus (Sarkar, *PCR Methods Applic.* 2, 318-322, 1993). Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region (Triglia *et al*., *Nucleic Acids Res. 16*, 8186, 1988). Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72 °C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA (Lagerstrom *et al., PCR Methods Applic. 1*, 111-119, 1991). In this method, multiple restriction enzyme digestions and ligations also can be used to place an engineered double-stranded sequence into an unknown fragment of the DNA molecule before performing PCR.

Another method which can be used to retrieve unknown sequences is that of Parker *et al*., *Nucleic Acids Res. 19*, 3055-3060, 1991). Additionally, PCR, nested primers, and PROMOTERFINDER libraries (CLONTECH, Palo Alto, Calif.) can be used to walk genomic DNA (CLONTECH, Palo Alto, Calif.). This process avoids the need to screen libraries and is useful in finding intron/exon junctions.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate software (*e.g.* GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

### Obtaining LT-GPCR Polypeptides

LT-GPCR polypeptides can be obtained, for example, by purification from human cells, by expression of LT-GPCR polynucleotides, or by direct chemical synthesis.

### Protein Purification

LT-GPCR polypeptides can be purified from any human cell which expresses the receptor, including host cells which have been transfected with LT-GPCR polynucleotides. Adult testis is a particularly useful source of LT-GPCR polypeptides. A purified LT-GPCR polypeptide is separated from other compounds which normally associate with the LT-GPCR polypeptide in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

LT-GPCR polypeptide can be conveniently isolated as a complex with its associated G protein, as described in the specific examples, below. A preparation of purified LT- GPCR polypeptides is at least 80% pure; preferably, the preparations are 90%, 95%, or 99% pure. Purity of the preparations can be assessed by any means known in the art, such as SDS-polyacrylamide gel electrophoresis.

### Expression of LT-GPCR Polynucleotides

To express an LT-GPCR polypeptide, an LT-GPCR polynucleotide can be inserted into an expression vector which contains the necessary elements for the transcription and translation of the inserted coding sequence. Methods which are well known to those skilled in the art can be used to construct expression vectors containing sequences encoding LT-GPCR polypeptides and appropriate transcriptional and translational control elements. These methods include *in vitro* recombinant DNA techniques, synthetic techniques, and *in vivo* genetic recombination. Such techniques are described, for example, in Sambrook *et al*. (1989) and in Ausubel *et al*., CURRENT PROTOCOLS IN MOLECULAR BIOLOGY, John Wiley & Sons, New York, N.Y., 1989.

A variety of expression vector/host systems can be utilized to contain and express sequences encoding an LT-GPCR polypeptide. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.*, baculovirus), plant cell systems transformed with virus expression vectors (*e.g.*, cauliflower mosaic virus, CaMV; tobacco mosaic virus, TMV) or with bacterial expression vectors *(e.g.,* Ti or pBR322 plasmids), or animal cell systems.

The control elements or regulatory sequences are those non-translated regions of the vector -- enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.*, heat shock, RUBISCO, and storage protein genes) or from plant viruses *(e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding an LT-GPCR polypeptide, vectors based on SV40 or EBV can be used with an appropriate selectable marker.

### Bacterial and Yeast Expression Systems

In bacterial systems, a number of expression vectors can be selected depending upon the use intended for the LT-GPCR polypeptide. For example, when a large quantity of an LT-GPCR polypeptide is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding the LT-GPCR polypeptide can be ligated into the vector in frame with sequences for the amino-terminal Met and the subsequent 7 residues of β-galactosidase so that a hybrid protein is produced. pIN vectors (Van Heeke & Schuster, *J. Biol. Chem. 264,* 5503-5509, 1989) or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

In the yeast *Saccharomyces cerevisiae,* a number of vectors containing constitutive or inducible promoters such as alpha factor, alcohol oxidase, and PGH can be used. For reviews, see Ausubel *et al*. (1989) and Grant *et al., Methods Enzymol. 153,* 516-544, 1987.

### Plant and Insect Expression Systems

If plant expression vectors are used, the expression of sequences encoding LT-GPCR polypeptides can be driven by any of a number of promoters. For example, viral promoters such as the 35S and 19S promoters of CaMV can be used alone or in combination with the omega leader sequence from TMV (Takamatsu, *EMBO J. 6,* 307-311, 1987). Alternatively, plant promoters such as the small subunit of RUBISCO or heat shock promoters can be used (Coruzzi *et al*., *EMBO J. 3*, 1671-1680, 1984; Broglie *et al*., *Science 224,* 838-843, 1984; Winter *et al*., *Results Probl. Cell Differ. 17*, 85-105, 1991). These constructs can be introduced into plant cells by direct DNA transformation or by pathogen-mediated transfection. Such techniques are described in a number of generally available reviews (*e*.*g*., Hobbs or Murray, in MCGRAW HILL YEARBOOK OF SCIENCE AND TECHNOLOGY, McGraw Hill, New York, N.Y., pp. 191-196, 1992).

An insect system also can be used to express an LT-GPCR polypeptide. For example, in one such system *Autographa californica* nuclear polyhedrosis virus (AcNPV) is used as a vector to express foreign genes in *Spodoptera frugiperda* cells or in *Trichoplusia* larvae. Sequences encoding LT-GPCR polypeptides can be cloned into a non-essential region of the virus, such as the polyhedrin gene, and placed under control of the polyhedrin promoter. Successful insertion of LT-GPCR polypeptides will render the polyhedrin gene inactive and produce recombinant virus lacking coat protein. The recombinant viruses can then be used to infect *S. frugiperda cells* or *Trichoplusia* larvae in which LT-GPCR polypeptides can be expressed (Engelhard *et al*., *Proc. Nat. Acad. Sci. 91*, 3224-3227, 1994).

### Mammalian Expression Systems

A number of viral-based expression systems can be used to express LT-GPCR polypeptides in mammalian host cells. For example, if an adenovirus is used as an expression vector, sequences encoding LT-GPCR polypeptides can be ligated into an adenovirus transcription/translation complex comprising the late promoter and tripartite leader sequence. Insertion in a non-essential E1 or E3 region of the viral genome can be used to obtain a viable virus which is capable of expressing an LT-GPCR polypeptide in infected host cells (Logan & Shenk, *Proc. Natl. Acad. Sci. 81,* 3655-3659, 1984). If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles).

Specific initiation signals also can be used to achieve more efficient translation of sequences encoding LT-GPCR polypeptides. Such signals include the ATG initiation codon and adjacent sequences. In cases where sequences encoding an LT-GPCR polypeptide, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic. The efficiency of expression can be enhanced by the inclusion of enhancers which are appropriate for the particular cell system which is used (see Scharf *et al., Results Probl. Cell Differ. 20,* 125-162, 1994).

### Host Cells

A host cell strain can be chosen for its ability to modulate the expression of the inserted sequences or to process the expressed LT-GPCR polypeptide in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities *(e.g.,* CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express LT-GPCR polypeptides can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced LT-GPCR sequences. Resistant clones of stably transformed cells can be proliferated using tissue culture techniques appropriate to the cell type. See, for example, ANIMAL CELL CULTURE, R.I. Freshney, ed., 1986.

Any number of selection systems can be used to recover transformed cell lines.

These include, but are not limited to, the herpes simplex virus thymidine kinase (Wigler *et al., Cell 11*, 223-32, 1977) and adenine phosphoribosyltransferase (Lowy *et al., Cell 22,* 817-23, 1980) genes which can be employed in *tk*^{*-*} or *aprt*^{*-*} cells, respectively. Also, antimetabolite, antibiotic, or herbicide resistance can be used as the basis for selection. For example, *dhfr* confers resistance to methotrexate (Wigler *et al*., *Proc. Natl. Acad. Sci.* 77, 3567-70, 1980), *npt* confers resistance to the aminoglycosides, neomycin and G-418 (Colbere-Garapin *et al*., *J. Mol. Biol. 150,* 1-14, 1981), and *als* and *pat* confer resistance to chlorsulfuron and phosphinotricin acetyltransferase, respectively (Murray, 1992, *supra*). Additional selectable genes have been described. For example, *trpB* allows cells to utilize indole in place of tryptophan, or *hisD*, which allows cells to utilize histinol in place of histidine (Hartman & Mulligan, *Proc. Natl. Acad. Sci. 85,* 8047-51, 1988). Visible markers such as anthocyanins, β-glucuronidase and its substrate GUS, and luciferase and its substrate luciferin, can be used to identify transformants and to quantify the amount of transient or stable protein expression attributable to a specific vector system (Rhodes *et al., Methods Mol. Biol. 55,* 121-131, 1995).

### Detecting Expression of LT-GPCR Polypeptides

Although the presence of marker gene expression suggests that the LT-GPCR polynucleotide is also present, its presence and expression may need to be confirmed. For example, if a sequence encoding an LT-GPCR polypeptide is inserted within a marker gene sequence, transformed cells containing sequences which encode an LT-GPCR polypeptide can be identified by the absence of marker gene function. Alternatively, a marker gene can be placed in tandem with a sequence encoding an LT-GPCR polypeptide under the control of a single promoter. Expression of the marker gene in response to induction or selection usually indicates expression of the LT-GPCR polynucleotide.

Alternatively, host cells which contain an LT-GPCR polynucleotide and which express an LT-GPCR polypeptide can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding an LT-GPCR polypeptide can be detected by DNA-DNA or DNA-RNA hybridization or amplification using probes or fragments or fragments of polynucleotides encoding an LT-GPCR polypeptide. Nucleic acid amplification-based assays involve the use of oligonucleotides selected from sequences encoding an LT-GPCR polypeptide to detect transformants which contain an LT-GPCR polynucleotide.

A variety of protocols for detecting and measuring the expression of an LT-GPCR polypeptide, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on an LT-GPCR polypeptide can be used, or a competitive binding assay can be employed. These and other assays are described in Hampton *et al*., SEROLOGICAL METHODS: A LABORATORY MANUAL, APS Press, St. Paul, Minn., 1990) and Maddox *et al*., *J. Exp. Med. 158,* 1211-1216, 1983).

A wide variety of labels and conjugation techniques are known by those skilled in the art and can be used in various nucleic acid and amino acid assays. Means for producing labeled hybridization or PCR probes for detecting sequences related to polynucleotides encoding LT-GPCR polypeptides include oligolabeling, nick translation, end-labeling, or PCR amplification using a labeled nucleotide. Alternatively, sequences encoding an LT-GPCR polypeptide can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

### Expression and Purification of LT-GPCR Polypeptides

Host cells transformed with nucleotide sequences encoding an LT-GPCR polypeptide can be cultured under conditions suitable for the expression and recovery of the polypeptide from cell culture. The polypeptide produced by a transformed cell can be secreted or contained intracellularly depending on the sequence and/or the vector used. As will be understood by those of skill in the art, expression vectors containing polynucleotides which encode LT-GPCR polypeptides can be designed to contain signal sequences which direct secretion of soluble LT-GPCR polypeptides through a prokaryotic or eukaryotic cell membrane or which direct the membrane insertion of membrane-bound LT-GPCR polypeptide.

As discussed above, other constructions can be used to join a sequence encoding an LT-GPCR polypeptide to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor Xa or enterokinase (Invitrogen, San Diego, CA) between the purification domain and the LT-GPCR polypeptide also can be used to facilitate purification. One such expression vector provides for expression of a fusion protein containing an LT-GPCR polypeptide and 6 histidine residues preceding a thioredoxin or an enterokinase cleavage site. The histidine residues facilitate purification by IMAC (immobilized metal ion affinity chromatography, as described in Porath *et al., Prot. Exp. Purif. 3, 263-281,* 1992), while the enterokinase cleavage site provides a means for purifying the LT-GPCR polypeptide from the fusion protein. Vectors which contain fusion proteins are disclosed in Kroll *et al., DNA. Cell Biol. 12,* 441-453, 1993.

### Chemical Synthesis

Sequences encoding an LT-GPCR polypeptide can be synthesized, in whole or in part, using chemical methods well known in the art (see Caruthers *et al., Nucl. Acids Res. Symp. Ser.* 215-223, 1980; Horn *et al. Nucl. Acids Res. Symp. Ser.* 225-232, 1980). Alternatively, an LT-GPCR polypeptide itself can be produced using chemical methods to synthesize its amino acid sequence, such as by direct peptide synthesis using solid-phase techniques (Merrifield, *J. Am. Chem. Soc. 85,* 2149-2154, 1963; *Roberge et al., Science 269,* 202-204, 1995). Protein synthesis can be performed using manual techniques or by automation. Automated synthesis can be achieved, for example, using Applied Biosystems 431A Peptide Synthesizer (Perkin Elmer). Optionally, fragments of LT-GPCR polypeptides can be separately synthesized and combined using chemical methods to produce a full-length molecule.

The newly synthesized peptide can be substantially purified by preparative high performance liquid chromatography (*e.g.*, Creighton, PROTEINS: STRUCTURES AND MOLECULAR PRINCIPLES, WH Freeman and Co., New York, N.Y., 1983). The composition of a synthetic LT-GPCR polypeptide can be confirmed by amino acid analysis or sequencing (*e.g.*, the Edman degradation procedure; *see* Creighton, *supra*). Additionally, any portion of the amino acid sequence of the LT-GPCR polypeptide can be altered during direct synthesis and/or combined using chemical methods with sequences from other proteins to produce a variant polypeptide or a fusion protein.

### Production of Altered LT-GPCR Polypeptides

As will be understood by those of skill in the art, it may be advantageous to produce LT-GPCR polypeptide-encoding nucleotide sequences possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences disclosed herein can be engineered using methods generally known in the art to alter LT-GPCR polypeptide-encoding sequences for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

### Antibodies

Any type of antibody known in the art can be generated to bind specifically to an epitope of an LT-GPCR polypeptide. "Antibody" as used herein includes intact immunoglobulin molecules, as well as fragments thereof, such as Fab, F(ab')₂, and Fv, which are capable of binding an epitope of an LT-GPCR polypeptide. Typically, at least 6, 8, 10, or 12 contiguous amino acids are required to form an epitope. However, epitopes which involve non-contiguous amino acids may require more, *e*.*g*., at least 15, 25, or 50 amino acids.

An antibody which specifically binds to an epitope of an LT-GPCR polypeptide can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the immunogen.

Typically, an antibody which specifically binds to an LT-GPCR polypeptide provides a detection signal at least 5-, 10-, or 20-fold higher than a detection signal provided with other proteins when used in an immunochemical assay. Preferably, antibodies which specifically bind to LT-GPCR polypeptides do not detect other proteins in immunochemical assays and can immunoprecipitate an LT-GPCR polypeptide from solution.

LT-GPCR polypeptides can be used to immunize a mammal, such as a mouse, rat, rabbit, guinea pig, monkey, or human, to produce polyclonal antibodies. If desired, an LT-GPCR polypeptide can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels *(e.g.,* aluminum hydroxide), and surface active substances (*e*.*g*. lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to an LT-GPCR polypeptide can be prepared using any technique which provides for the production of antibody molecules by continuous cell lines in culture. These techniques include, but are not limited to, the hybridoma technique, the human B-cell hybridoma technique, and the EBV-hybridoma technique (Kohler *et al*., *Nature 256*, 495-497, 1985; Kozbor *et al*., *J. Immunol. Methods 81,* 31-42, 1985; Cote *et al*., *Proc. Natl. Acad. Sci. 80,* 2026-2030, 1983; Cole *et al*., *Mol. Cell Biol. 62*, 109-120, 1984).

In addition, techniques developed for the production of "chimeric antibodies," the splicing of mouse antibody genes to human antibody genes to obtain a molecule with appropriate antigen specificity and biological activity, can be used (Morrison *et al*., *Proc. Natl. Acad. Sci. 81,* 6851-6855, 1984; Neuberger *et al*., *Nature 312,* 604-608, 1984; Takeda. *et al*., *Nature 314,* 452-454, 1985). Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Alternatively, humanized antibodies can be produced using recombinant methods, as described in GB2188638B. Antibodies which specifically bind to an LT-GPCR polypeptide can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to LT-GPCR polypeptides. Antibodies with related specificity, but of distinct idiotypic composition, can be generated by chain shuffling from random combinatorial immunoglobin libraries (Burton, *Proc. Natl. Acad. Sci. 88*, 11120-23, 1991).

Single-chain antibodies also can be constructed using a DNA amplification method, such as PCR, using hybridoma cDNA as a template (Thirion *et al*., 1996, *Eur. J. Cancer Prev. 5*, 507-11). Single-chain antibodies can be mono- or bispecific, and can be bivalent or tetravalent. Construction of tetravalent, bispecific single-chain antibodies is taught, for example, in Coloma & Morrison, 1997, *Nat. Biotechnol. 15,* 159-63. Construction of bivalent, bispecific single-chain antibodies is taught in Mallender & Voss, 1994, *J. Biol. Chem. 269,* 199-206.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (Verhaar *et al*., 1995, *Int. J. Cancer 61,* 497-501; Nicholls *et al*., 1993, *J Immunol. Meth. 165,* 81-91).

Antibodies which specifically bind to LT-GPCR polypeptides also can be produced by inducing *in vivo* production in the lymphocyte population or by screening immunoglobulin libraries or panels of highly specific binding reagents as disclosed in the literature (Orlandi *et al*., *Proc. Natl. Acad. Sci.* 86, 3833-3837, 1989; Winter *et al*., *Nature 349,* 293-299, 1991).

Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which an LT-GPCR polypeptide is bound. The bound antibodies can then be eluted from the column using a buffer with a high salt concentration.

### Antisense Oligonucleotides

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of LT-GPCR gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters. *See* Brown, *Meth. Mol. Biol. 20,* 1-8, 1994; Sonveaux, *Meth. Mol. Biol. 26,* 1-72, 1994; Uhlmann *et al., Chem. Rev. 90, 543-583,* 1990.

Modifications of LT-GPCR gene expression can be obtained by designing antisense oligonucleotides which will form duplexes to the control, 5', or regulatory regions of the LT-GPCR gene. Oligonucleotides derived from the transcription initiation site, *e.g.,* between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature (*e.g.*, Gee *et al*., in Huber & Carr, MOLECULAR AND IMMUNOLOGIC APPROACHES, Futura Publishing Co., Mt. Kisco, N.Y., 1994). An antisense oligonucleotide also can be designed to block translation of mRNA by preventing the transcript from binding to ribosomes.

Precise complementarity is not required for successful complex formation between an antisense oligonucleotide and the complementary sequence of an LT-GPCR polynucleotide. Antisense oligonucleotides which comprise, for example, 2, 3, 4, or 5 or more stretches of contiguous nucleotides which are precisely complementary to an LT-GPCR polynucleotide, each separated by a stretch of contiguous nucleotides which are not complementary to adjacent LT-GPCR nucleotides, can provide sufficient targeting specificity for LT-GPCR mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular LT-GPCR polynucleotide sequence.

Antisense oligonucleotides can be modified without affecting their ability to hybridize to an LT-GPCR polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art. *See, e.g.*, Agrawal *et al., Trends Biotechnol. 10,* 152-158, 1992; Uhlmann *et al., Chem. Rev. 90*, 543-584, 1990; Uhlmann *et al., Tetrahedron. Lett. 215*, 3539-3542, 1987.

### Ribozymes

Ribozymes are RNA molecules with catalytic activity. *See, e.g.,* Cech, *Science 236*, 1532-1539; 1987; Cech, *Ann. Rev. Biochem. 59,* 543-568; 1990, Cech, *Curr. Opin. Struct. Biol. 2,* 605-609; 1992, Couture & Stinchcomb, *Trends Genet. 12*, 510-515, 1996. Ribozymes can be used to inhibit gene function by cleaving an RNA sequence, as is known in the art (*e*.*g*., Haseloff *et al*., U.S. Patent 5,641,673). The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences.

The coding sequence of an LT-GPCR polynucleotide, such as the nucleotide sequence shown in SEQ ID NO:1, can be used to generate ribozymes which will specifically bind to mRNA transcribed from the LT-GPCR polynucleotide. Methods of designing and constructing ribozymes which can cleave other RNA molecules in trans in a highly sequence specific manner have been developed and described in the art (*see* Haseloff *et al*. *Nature 334,* 585-591, 1988). For example, the cleavage activity of ribozymes can be targeted to specific RNAs by engineering a discrete "hybridization" region into the ribozyme. The hybridization region contains a sequence complementary to the target RNA and thus specifically hybridizes with the target (see, for example, Gerlach *et al*., EP 321,201).

Specific ribozyme cleavage sites within an LT-GPCR RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate LT-GPCR RNA targets also can be evaluated by testing accessibility to hybridization with complementary oligonucleotides using ribonuclease protection assays. The nucleotide sequence shown in SEQ ID NO:1 and its complement provides a source of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease LT-GPCR expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells.

As taught in Haseloff *et al*., U.S. Patent 5,641,673, ribozymes can be engineered so that ribozyme expression will occur in response to factors which induce expression of a target gene. Ribozymes also can be engineered to provide an additional level of regulation, so that destruction of mRNA occurs only when both a ribozyme and a target gene are induced in the cells.

### Screening Methods

The invention provides assays for screening test compounds which bind to or modulate the activity of an LT-GPCR polypeptide or an LT-GPCR polynucleotide. A test compound preferably binds to an LT-GPCR polypeptide or polynucleotide. More preferably, a test compound decreases or increases a biological effect mediated via human LT-GPCR by at least about 10, preferably about 50, more preferably about 75, 90, or 100% relative to the absence of the test compound.

### Test Compounds

Test compounds can be pharmacologic agents already known in the art or can be compounds previously unknown to have any pharmacological activity. The compounds can be naturally occurring or designed in the laboratory. They can be isolated from microorganisms, animals, or plants, and can be produced recombinantly, or synthesized by chemical methods known in the art. If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. *See* Lam, *Anticancer Drug Des. 12*, 145, 1997.

Methods for the synthesis of molecular libraries are well known in the art (*see,* for example, DeWitt *et al*., *Proc. Natl. Acad. Sci. U.S.A. 90*, 6909, 1993; Erb *et al*. *Proc. Natl. Acad. Sci. U.S.A. 91,* 11422, 1994; Zuckermann *et al*., *J. Med. Chem. 37*, 2678, 1994; Cho *et al*., *Science 261,* 1303, 1993; Carell *et al*., *Angew. Chem. Int. Ed. Engl. 33*, 2059, 1994; Carell *et al*., *Angew. Chem. Int. Ed. Engl. 33,* 2061; Gallop *et al*., *J. Med. Chem. 37*, 1233, 1994). Libraries of compounds can be presented in solution (*see, e.g.,* Houghten, *Biotechniques 13,* 412-421, 1992), or on beads (Lam, *Nature 354*, 82-84, 1991), chips (Fodor, *Nature 364,* 555-556, 1993), bacteria or spores (Ladner, U.S. Patent 5,223,409), plasmids (Cull *et al*., *Proc. Natl. Acad Sci. U.S.A*. 89, 1865-1869, 1992), or phage (Scott & Smith, *Science 249,* 386-390, 1990; Devlin, *Science 249,* 404-406, 1990); Cwirla *et al., Proc. Natl. Acad. Sci. 97,* 6378-6382, 1990; Felici, *J. Mol. Biol*. *222*, 301-310, 1991; and Ladner, U.S. Patent 5,223,409).

### High Throughput Screening

Test compounds can be screened for the ability to bind to LT-GPCR polypeptides or polynucleotides or to affect LT-GPCR activity or LT-GPCR gene expression using high throughput screening. Using high throughput screening, many discrete compounds can be tested in parallel so that large numbers of test compounds can be quickly screened. The most widely established techniques utilize 96-well microtiter plates. The wells of the microtiter plates typically require assay volumes that range from 50 to 500 1. In addition to the plates, many instruments, materials, pipettors, robotics, plate washers, and plate readers are commercially available to fit the 96-well format.

Alternatively, "free format assays," or assays that have no physical barrier between samples, can be used. For example, an assay using pigment cells (melanocytes) in a simple homogeneous assay for combinatorial peptide libraries is described by Jayawickreme *et al., Proc. Natl. Acad. Sci. U.S.A. 19,* 1614-18 (1994). The cells are placed under agarose in petri dishes, then beads that carry combinatorial compounds are placed on the surface of the agarose. The combinatorial compounds are partially released the compounds from the beads. Active compounds can be visualized as dark pigment areas because, as the compounds diffuse locally into the gel matrix, the active compounds cause the cells to change colors.

Another example of a free format assay is described by Chelsky, "Strategies for Screening Combinatorial Libraries: Novel and Traditional Approaches," reported at the First Annual Conference of The Society for Biomolecular Screening in Philadelphia, Pa. (Nov. 7-10, 1995). Chelsky placed a simple homogenous enzyme assay for carbonic anhydrase inside an agarose gel such that the enzyme in the gel would cause a color change throughout the gel. Thereafter, beads carrying combinatorial compounds via a photolinker were placed inside the gel and the compounds were partially released by UV-light. Compounds that inhibited the enzyme were observed as local zones of inhibition having less color change.

Yet another example is described by Salmon *et al., Molecular Diversity 2*, 57-63 (1996). In this example, combinatorial libraries were screened for compounds that had cytotoxic effects on cancer cells growing in agar.

Another high throughput screening method is described in Beutel *et al*., U.S. Patent 5,976,813. In this method, test samples are placed in a porous matrix. One or more assay components are then placed within, on top of, or at the bottom of a matrix such as a gel, a plastic sheet, a filter, or other form of easily manipulated solid support. When samples are introduced to the porous matrix they diffuse sufficiently slowly, such that the assays can be performed without the test samples running together.

### Binding Assays

For binding assays, the test compound is preferably a small molecule which binds to and occupies the active site of the LT-GPCR polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known LT-GPCRs and analogues or derivatives thereof. Natural ligands of LT-GPCRs include adrenomedullin, amylin, calcitonin gene related protein (CGRP), calcitonin, anandamide, serotonin, histamine, adrenalin, noradrenalin, platelet activating factor, thrombin, C5a, bradykinin, and chemokines.

In binding assays, either the test compound or the LT-GPCR polypeptide can comprise a detectable label, such as a fluorescent, radioisotopic, chemiluminescent, or enzymatic label, such as horseradish peroxidase, alkaline phosphatase, or luciferase. Detection of a test compound which is bound to the LT-GPCR polypeptide can then be accomplished, for example, by direct counting of radioemmission, by scintillation counting, or by determining conversion of an appropriate substrate to a detectable product.

Alternatively, binding of a test compound to an LT-GPCR polypeptide can be determined without labeling either of the interactants. For example, a microphysiometer can be used to detect binding of a test compound with an LT-GPCR polypeptide. A microphysiometer (*e*.*g*., Cytosensor^{™}) is an analytical instrument that measures the rate at which a cell acidifies its environment using a light-addressable potentiometric sensor (LAPS). Changes in this acidification rate can be used as an indicator of the interaction between a test compound and an LT-GPCR polypeptide (McConnell *et al., Science 257*, 1906-1912, 1992).

Determining the ability of a test compound to bind to an LT-GPCR polypeptide also can be accomplished using a technology such as real-time Bimolecular Interaction Analysis (BIA) (Sjolander & Urbaniczky, *Anal. Chem. 63,* 2338-2345, 1991, and Szabo *et al*., *Curr. Opin. Struct. Biol. 5*, 699-705, 1995). BIA is a technology for studying biospecific interactions in real time, without labeling any of the interactants (*e.g.,* BIAcore^{™}). Changes in the optical phenomenon surface plasmon resonance (SPR) can be used as an indication of real-time reactions between biological molecules.

In yet another aspect of the invention, an LT-GPCR polypeptide can be used as a "bait protein" in a two-hybrid assay or three-hybrid assay (see, *e.g.*, U.S. Patent 5,283,317; Zervos *et al*., *Cell* 72, 223-232, 1993; Madura *et al*., *J. Biol. Chem. 268*, 12046-12054, 1993; Bartel *et al*., *Biotechniques 14*, 920-924, 1993; Iwabuchi *et al*., *Oncogene 8,* 1693-1696, 1993; and Brent WO94/10300), to identify other proteins which bind to or interact with the LT-GPCR polypeptide and modulate its activity.

The two-hybrid system is based on the modular nature of most transcription factors, which consist of separable DNA-binding and activation domains. Briefly, the assay utilizes two different DNA constructs. For example, in one construct, polynucleotide encoding an LT-GPCR polypeptide can be fused to a polynucleotide encoding the DNA binding domain of a known transcription factor (*e*.*g*., GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (*e.g.*, LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with the LT-GPCR polypeptide.

It may be desirable to immobilize either the LT-GPCR polypeptide (or polynucleotide) or the test compound to facilitate separation of bound from unbound forms of one or both of the interactants, as well as to accommodate automation of the assay. Thus, either the LT-GPCR polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach the LT-GPCR polypeptide (or polynucleotide) or test compound to a solid support, including use of covalent and non-covalent linkages, passive absorption, or pairs of binding moieties attached respectively to the polypeptide (or polynucleotide) or test compound and the solid support. Test compounds are preferably bound to the solid support in an array, so that the location of individual test compounds can be tracked. Binding of a test compound to an LT-GPCR polypeptide (or polynucleotide) can be accomplished in any vessel suitable for containing the reactants. Examples of such vessels include microtiter plates, test tubes, and microcentrifuge tubes.

In one embodiment, the LT-GPCR polypeptide is a fusion protein comprising a domain that allows the LT-GPCR polypeptide to be bound to a solid support. For example, glutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed LT-GPCR polypeptide; the mixture is then incubated under conditions conducive to complex formation (*e.g.*, at physiological conditions for salt and pH). Following incubation, the beads or microtiter plate wells are washed to remove any unbound components. Binding of the interactants can be determined either directly or indirectly, as described above. Alternatively, the complexes can be dissociated from the solid support before binding is determined.

Other techniques for immobilizing proteins or polynucleotides on a solid support also can be used in the screening assays of the invention. For example, either an LT-GPCR polypeptide (or polynucleotide) or a test compound can be immobilized utilizing conjugation of biotin and streptavidin. Biotinylated LT-GPCR polypeptides (or polynucleotides) or test compounds can be prepared from biotin-NHS(N-hydroxysuccinimide) using techniques well known in the art (*e.g.*, biotinylation kit, Pierce Chemicals, Rockford, Ill.) and immobilized in the wells of streptavidin-coated 96 well plates (Pierce Chemical). Alternatively, antibodies which specifically bind to an LT-GPCR polypeptide, polynucleotide, or a test compound, but which do not interfere with a desired binding site, such as the active site of the LT-GPCR polypeptide, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to the LT-GPCR polypeptide or test compound, enzyme-linked assays which rely on detecting an activity of the LT-GPCR polypeptide, and SDS gel electrophoresis under non-reducing conditions.

Screening for test compounds which bind to an LT-GPCR polypeptide or polynucleotide also can be carried out in an intact cell. Any cell which comprises an LT-GPCR polypeptide or polynucleotide can be used in a cell-based assay system. An LT-GPCR polynucleotide can be naturally occurring in the cell or can be introduced using techniques such as those described above. Binding of the test compound to an LT-GPCR polypeptide or polynucleotide is determined as described above.

### Functional Assays

Test compounds can be tested for the ability to increase or decrease a biological effect of an LT-GPCR polypeptide. Such biological effects can be determined using the functional assays described in the specific examples, below. Functional assays can be carried out after contacting either a purified LT-GPCR polypeptide, a cell membrane preparation, or an intact cell with a test compound. A test compound which decreases a functional activity of an LT-GPCR by at least about 10, preferably about 50, more preferably about 75, 90, or 100% is identified as a potential agent for decreasing LT-GPCR activity. A test compound which increases LT-GPCR activity by at least about 10, preferably about 50, more preferably about 75, 90, or 100% is identified as a potential agent for increasing LT-GPCR activity.

One such screening procedure involves the use of melanophores which are transfected to express an LT-GPCR polypeptide. Such a screening technique is described in WO 92/01810 published Feb. 6, 1992. Thus, for example, such an assay may be employed for screening for a compound which inhibits activation of the receptor polypeptide by contacting the melanophore cells which comprise the receptor with both the receptor ligand and a test compound to be screened. Inhibition of the signal generated by the ligand indicates that a test compound is a potential antagonist for the receptor, *i.e.*, inhibits activation of the receptor. The screen may be employed for identifying a test compound which activates the receptor by contacting such cells with compounds to be screened and determining whether each test compound generates a signal, *i*.*e*., activates the receptor.

Other screening techniques include the use of cells which express a human LT-GPCR polypeptide (for example, transfected CHO cells) in a system which measures extracellular pH changes caused by receptor activation (*see, e.g., Science 246,* 181-296, 1989). For example, test compounds may be contacted with a cell which expresses a human LT-GPCR polypeptide and a second messenger response, *e.g.,* signal transduction or pH changes, can be measured to determine whether the test compound activates or inhibits the receptor.

Another such screening technique involves introducing RNA encoding a human LT-GPCR polypeptide into *Xenopus* oocytes to transiently express the receptor. The transfected oocytes can then be contacted with the receptor ligand and a test compound to be screened, followed by detection of inhibition or activation of a calcium signal in the case of screening for test compounds which are thought to inhibit activation of the receptor.

Another screening technique involves expressing a human LT-GPCR polypeptide in cells in which the receptor is linked to a phospholipase C or D. Such cells include endothelial cells, smooth muscle cells, embryonic kidney cells, etc. The screening may be accomplished as described above by quantifying the degree of activation of the receptor from changes in the phospholipase activity.

Details of functional assays such as those described above are provided in the specific examples, below.

### LT-GPCR Gene Expression

In another embodiment, test compounds which increase or decrease LT-GPCR gene expression are identified. An LT-GPCR polynucleotide is contacted with a test compound, and the expression of an RNA or polypeptide product of the LT-GPCR polynucleotide is determined. The level of expression of appropriate mRNA or polypeptide in the presence of the test compound is compared to the level of expression of mRNA or polypeptide in the absence of the test compound. The test compound can then be identified as a modulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of LT-GPCR mRNA or polypeptide expression in the cells can be determined by methods well known in the art for detecting mRNA or polypeptide. Either qualitative or quantitative methods can be used. The presence of polypeptide products of an LT-GPCR polynucleotide can be determined, for example, using a variety of techniques known in the art, including immunochemical methods such as radioimmunoassay, Western blotting, and immunohistochemistry. Alternatively, polypeptide synthesis can be determined *in vivo,* in a cell culture, or in an *in vitro* translation system by detecting incorporation of labeled amino acids into an LT-GPCR polypeptide.

Such screening can be carried out either in a cell-free assay system or in an intact cell. Any cell which expresses an LT-GPCR polynucleotide can be used in a cell-based assay system. The LT-GPCR polynucleotide can be naturally occurring in the cell or can be introduced using techniques such as those described above. Either a primary culture or an established cell line, such as CHO or human embryonic kidney 293 cells, can be used.

### Pharmaceutical Compositions

The invention also provides pharmaceutical compositions which can be administered to a patient to achieve a therapeutic effect. Pharmaceutical compositions of the invention can comprise, for example, an LT-GPCR polypeptide, LT-GPCR polynucleotide, antibodies which specifically bind to an LT-GPCR polypeptide, or mimetics, agonists, antagonists, or inhibitors of an LT-GPCR polypeptide activity. The compositions can be administered alone or in combination with at least one other agent, such as stabilizing compound, which can be administered in any sterile, biocompatible pharmaceutical carrier, including, but not limited to, saline, buffered saline, dextrose, and water. The compositions can be administered to a patient alone, or in combination with other agents, drugs or hormones.

In addition to the active ingredients, these pharmaceutical compositions can contain suitable pharmaceutically-acceptable carriers comprising excipients and auxiliaries which facilitate processing of the active compounds into preparations which can be used pharmaceutically. Pharmaceutical compositions of the invention can be administered by any number of routes including, but not limited to, oral, intravenous, intramuscular, intra-arterial, intramedullary, intrathecal, intraventricular, transdermal, subcutaneous, intraperitoneal, intranasal, parenteral, topical, sublingual, or rectal means. Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents can be added, such as the cross-linked polyvinyl pyrrolidone, agar, alginic acid, or a salt thereof, such as sodium alginate.

Dragee cores can be used in conjunction with suitable coatings, such as concentrated sugar solutions, which also can contain gum arabic, talc, polyvinylpyrrolidone, carbopol gel, polyethylene glycol, and/or titanium dioxide, lacquer solutions, and suitable organic solvents or solvent mixtures. Dyestuffs or pigments can be added to the tablets or dragee coatings for product identification or to characterize the quantity of active compound, *i*.*e*., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Pharmaceutical formulations suitable for parenteral administration can be formulated in aqueous solutions, preferably in physiologically compatible buffers such as Hanks' solution, Ringer's solution, or physiologically buffered saline. Aqueous injection suspensions can contain substances which increase the viscosity of the suspension, such as sodium carboxymethyl cellulose, sorbitol, or dextran. Additionally, suspensions of the active compounds can be prepared as appropriate oily injection suspensions. Suitable lipophilic solvents or vehicles include fatty oils such as sesame oil, or synthetic fatty acid esters, such as ethyl oleate or triglycerides, or liposomes. Non-lipid polycationic amino polymers also can be used for delivery. Optionally, the suspension also can contain suitable stabilizers or agents which increase the solubility of the compounds to allow for the preparation of highly concentrated solutions. For topical or nasal administration, penetrants appropriate to the particular barrier to be permeated are used in the formulation. Such penetrants are generally known in the art.

The pharmaceutical compositions of the present invention can be manufactured in a manner that is known in the art, *e.g.*, by means of conventional mixing, dissolving, granulating, dragee-making, levigating, emulsifying, encapsulating, entrapping, or lyophilizing processes. The pharmaceutical composition can be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation can be a lyophilized powder which can contain any or all of the following: 1-50 mM histidine, 0.1%-2% sucrose, and 2-7% mannitol, at a pH range of 4.5 to 5.5, that is combined with buffer prior to use.

Further details on techniques for formulation and administration can be found in the latest edition of REMINGTON'S PHARMACEUTICAL SCIENCES (Maack Publishing Co., Easton, Pa.). After pharmaceutical compositions have been prepared, they can be placed in an appropriate container and labeled for treatment of an indicated condition. Such labeling would include amount, frequency, and method of administration.

### Therapeutic Indications and Methods

GPCRs are ubiquitous in the mammalian host and are responsible for many biological functions, including many pathologies. Accordingly, it is desirable to find compounds and drugs which stimulate a GPCR on the one hand and which can inhibit the function of a GPCR on the other hand. For example, compounds which activate a GPCR may be employed for therapeutic purposes, such as the treatment of asthma, Parkinson's disease, acute heart failure, urinary retention, and osteoporosis. In particular, compounds which activate GPCRs are useful in treating various cardiovascular ailments such as caused by the lack of pulmonary blood flow or hypertension. In addition these compounds may also be used in treating various physiological disorders relating to abnormal control of fluid and electrolyte homeostasis and in diseases associated with abnormal angiotensin-induced aldosterone secretion.

In general, compounds which inhibit activation of a GPCR can be used for a variety of therapeutic purposes, for example, for the treatment of hypotension and/or hypertension, angina pectoris, myocardial infarction, ulcers, asthma, allergies, benign prostatic hypertrophy, and psychotic and neurological disorders including schizophrenia, manic excitement, depression, delirium, dementia or severe mental retardation, dyskinesias, such as Huntington's disease or Tourett's syndrome, among others. Compounds which inhibit GPCRs also are useful in reversing endogenous anorexia, in the control of bulimia, and in treating various cardiovascular ailments such as caused by excessive pulmonary blood flow or hypotension.

Regulation of LT-GPCR can be used to regulate exocytosis. For example, antagonists of LT-GPCR can be used to decrease exocytosis of hormones, for example, from benign tumors of the endocrine glands, such as pituitary adenoma of acromegaly, toxic adenoma which produces hyperthyroidism, parathyroid adenoma of hyperparathyroidism, insulin-secreting islet cell adenoma of the pancreas, benign pheochromocytoma, and adrenal cortical adenoma of Cushing's syndrome, malignant hormone-secreting tumors of the anterior pituitary and thyroid, cancers of the parathyroids, islet cells, adrenal medulla, and adrenal cortex. Other hyperfunctional states, such as Graves disease, toxic diffuse goiter, Cushing's syndrome not associated with adrenal tumor, Zollinger-Ellison syndrome, hyperthyroidism associated with TSH overproduction by pituitary tumors, also can be treated.

Agonists of LT-GPCR can be used to treat disorders characterized by low endocrine function, such as hypothyroidism of Hashimoto's thyroiditis, hypoparathyroidism, Addison's disease, diabetes mellitus, tumors which destroy or impair the function of an endocrine gland, including metastasizing cancers such as breast cancer and lymphoma, chromophobe adenoma, and hypophysitis.

Defects in neurotransmission also can be treated by regulating levels of LT-GPCR in the central and peripheral nervous systems.

This invention further pertains to the use of novel agents identified by the screening assays described above. Accordingly, it is within the scope of this invention to use a test compound identified as described herein in an appropriate animal model. For example, an agent identified as described herein (*e*.*g*., a modulating agent, an antisense nucleic acid molecule, a specific antibody, ribozyme, or an LT-GPCR polypeptide binding molecule) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

A reagent which affects LT-GPCR activity can be administered to a human cell, either *in vitro* or *in vivo*, to reduce LT-GPCR activity. The reagent preferably binds to an expression product of a human LT-GPCR gene. If the expression product is a protein, the reagent is preferably an antibody. For treatment of human cells *ex vivo,* an antibody can be added to a preparation of stem cells which have been removed from the body. The cells can then be replaced in the same or another human body, with or without clonal propagation, as is known in the art.

In one embodiment, the reagent is delivered using a liposome. Preferably, the liposome is stable in the animal into which it has been administered for at least about 30 minutes, more preferably for at least about 1 hour, and even more preferably for at least about 24 hours. A liposome comprises a lipid composition that is capable of targeting a reagent, particularly a polynucleotide, to a particular site in an animal, such as a human. Preferably, the lipid composition of the liposome is capable of targeting to a specific organ of an animal, such as the lung, liver, spleen, heart brain, lymph nodes, and skin.

A liposome useful in the present invention comprises a lipid composition that is capable of fusing with the plasma membrane of the targeted cell to deliver its contents to the cell. Preferably, the transfection efficiency of a liposome is about 0.5 µg of DNA per 16 nmole of liposome delivered to about 10⁶ cells, more preferably about 1.0 µg of DNA per 16 nmole of liposome delivered to about 10⁶ cells, and even more preferably about 2.0 µg of DNA per 16 nmol of liposome delivered to about 10⁶ cells. Preferably, a liposome is between about 100 and 500 nm, more preferably between about 150 and 450 nm, and even more preferably between about 200 and 400 nm in diameter.

Suitable liposomes for use in the present invention include those liposomes standardly used in, for example, gene delivery methods known to those of skill in the art. More preferred liposomes include liposomes having a polycationic lipid composition and/or liposomes having a cholesterol backbone conjugated to polyethylene glycol. Optionally, a liposome comprises a compound capable of targeting the liposome to a tumor cell, such as a tumor cell ligand exposed on the outer surface of the liposome.

Complexing a liposome with a reagent such as an antisense oligonucleotide or ribozyme can be achieved using methods which are standard in the art (see, for example, U.S. Patent 5,705,151). Preferably, from about 0.1 µg to about 10 µg of polynucleotide is combined with about 8 nmol of liposomes, more preferably from about 0.5 µg to about 5 µg of polynucleotides are combined with about 8 nmol liposomes, and even more preferably about 1.0 µg of polynucleotides is combined with about 8 nmol liposomes.

In another embodiment, antibodies can be delivered to specific tissues *in vivo* using receptor-mediated targeted delivery. Receptor-mediated DNA delivery techniques are taught in, for example, *Findeis et al. Trends in Biotechnol. 11,* 202-05 (1993); Chiou *et al*., GENE THERAPEUTICS: METHODS AND APPLICATIONS OF DIRECT GENE TRANSFER (J.A. Wolff, ed.) (1994); Wu & Wu, *J. Biol. Chem.* 263, 621-24 (1988); Wu *et al*., *J. Biol. Chem. 269,* 542-46 (1994); Zenke *et al*., *Proc. Natl. Acad. Sci. U.S.A. 87*, 3655-59 (1990); Wu *et al*., *J. Biol. Chem. 266,* 338-42 (1991).

### Determination of a Therapeutically Effective Dose

The determination of a therapeutically effective dose is well within the capability of those skilled in the art. A therapeutically effective dose refers to that amount of active ingredient which increases or decreases LT-GPCR activity relative to the LT-GPCR activity which occurs in the absence of the therapeutically effective dose.

For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, *e.g.*, ED₅₀ (the dose therapeutically effective in 50% of the population) and LD₅₀ (the dose lethal to 50% of the population), can be determined by standard pharmaceutical procedures in cell cultures or experimental animals. The dose ratio of toxic to therapeutic effects is the therapeutic index, and it can be expressed as the ratio, LD₅₀/ED₅₀.

Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED₅₀ with little or no toxicity. The dosage varies within this range depending upon the dosage form employed, sensitivity of the patient, and the route of administration.

The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun," and DEAE- or calcium phosphate-mediated transfection.

Effective *in vivo* dosages of an antibody are in the range of about 5 µg to about 50 µg/kg, about 50 µg to about 5 mg/kg, about 100 µg to about 500 µg/kg of patient body weight, and about 200 to about 250 µg/kg of patient body weight. For administration of polynucleotides encoding single-chain antibodies, effective *in vivo* dosages are in the range of about 100 ng to about 200 ng, 500 ng to about 50 mg, about 1 µg to about 2 mg, about 5 µg to about 500 µg, and about 20 µg to about 100 µg of DNA.

If the expression product is mRNA, the reagent is preferably an antisense oligonucleotide or a ribozyme. Polynucleotides which express antisense oligonucleotides or ribozymes can be introduced into cells by a variety of methods, as described above.

Preferably, a reagent reduces expression of an LT-GPCR gene or the activity of an LT-GPCR polypeptide by at least about 10, preferably about 50, more preferably about 75, 90, or 100% relative to the absence of the reagent. The effectiveness of the mechanism chosen to decrease the level of expression of an LT-GPCR gene or the activity of an LT-GPCR polypeptide can be assessed using methods well known in the art, such as hybridization of nucleotide probes to LT-GPCR-specific mRNA, quantitative RT-PCR, immunologic detection of an LT-GPCR polypeptide, or measurement of LT-GPCR activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects.

Any of the therapeutic methods described above can be applied to any subject in need of such therapy, including, for example, mammals such as dogs, cats, cows, horses, rabbits, monkeys, and most preferably, humans.

### Diagnostic Methods

GPCRs also can be used in diagnostic assays for detecting diseases and abnormalities or susceptibility to diseases and abnormalities related to the presence of mutations in the nucleic acid sequences which encode a GPCR. Such diseases, by way of example, are related to cell transformation, such as tumors and cancers, and various cardiovascular disorders, including hypertension and hypotension, as well as diseases arising from abnormal blood flow, abnormal angiotensin-induced aldosterone secretion, and other abnormal control of fluid and electrolyte homeostasis.

Differences can be determined between the cDNA or genomic sequence encoding a GPCR in individuals afflicted with a disease and in normal individuals. If a mutation is observed in some or all of the afflicted individuals but not in normal individuals, then the mutation is likely to be the causative agent of the disease.

Sequence differences between a reference gene and a gene having mutations can be revealed by the direct DNA sequencing method. In addition, cloned DNA segments can be employed as probes to detect specific DNA segments. The sensitivity of this method is greatly enhanced when combined with PCR. For example, a sequencing primer can be used with a double-stranded PCR product or a single-stranded template molecule generated by a modified PCR. The sequence determination is performed by conventional procedures using radiolabeled nucleotides or by automatic sequencing procedures using fluorescent tags.

Genetic testing based on DNA sequence differences can be carried out by detection of alteration in electrophoretic mobility of DNA fragments in gels with or without denaturing agents. Small sequence deletions and insertions can be visualized, for example, by high resolution gel electrophoresis. DNA fragments of different sequences can be distinguished on denaturing formamide gradient gels in which the mobilities of different DNA fragments are retarded in the gel at different positions according to their specific melting or partial melting temperatures (*see, e*.*g*., Myers *et al., Science 230,* 1242, 1985). Sequence changes at specific locations can also be revealed by nuclease protection assays, such as RNase and S 1 protection or the chemical cleavage method (*e*.*g*., Cotton *et al., Proc. Natl. Acad Sci. USA 85,* 4397-4401, 1985). Thus, the detection of a specific DNA sequence can be performed by methods such as hybridization, RNase protection, chemical cleavage, direct DNA sequencing or the use of restriction enzymes and Southern blotting of genomic DNA. In addition to direct methods such as gel-electrophoresis and DNA sequencing, mutations can also be detected by *in situ* analysis.

Altered levels of a GPCR also can be detected in various tissues. Assays used to detect levels of the receptor polypeptides in a body sample, such as blood or a tissue biopsy, derived from a host are well known to those of skill in the art and include radioimmunoassays, competitive binding assays, Western blot analysis, and ELISA assays.

### EXAMPLE 1

### Detection of LT-GPCR protein activity

The polynucleotide of SEQ ID NO: 1 is inserted into the expression vector pCEV4 and the expression vector pCEV4-LT-GPCR polypeptide obtained is transfected into human embryonic kidney 293 cells. The cells are scraped from a culture flask into 5 ml of Tris HCl, 5 mM EDTA, pH 7.5, and lysed by sonication. Cell lysates are centrifuged at 1000 rpm for 5 minutes at 4 °C. The supernatant is centrifuged at 30,000 x g for 20 minutes at 4 °C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 µg/ml phosphoramidon. Optimal membrane suspension dilutions, defined as the protein concentration required to bind less than 10 % of an added radioligand, i.e. ¹²⁵I-Iabeled Alpha-latrotoxin (ALX), are added to 96-well polypropylene microtiter plates containing ligand, non-labeled peptides, and binding buffer to a final volume of 250 µl.

In equilibrium saturation binding assays, membrane preparations are incubated in the presence of increasing concentrations (0.1 nM to 4 nM) of ¹²⁵I ligand.

Binding reaction mixtures are incubated for one hour at 30 °C. The reaction is stopped by filtration through GF/B filters treated with 0.5% polyethyleneimine, using a cell harvester. Radioactivity is measured by scintillation counting, and data are analyzed by a computerized non-linear regression program. Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. The LT-GPCR protein activity of the polypeptide comprising the amino acid sequence of SEQ ID NO: 2 is demonstrated.

### EXAMPLE 2

### Radioligand binding assays

Human embryonic kidney 293 cells transfected with a polynucleotide which expresses human latrophilin-like GPCR are scraped from a culture flask into 5 ml of Tris HCl, 5 mM EDTA, pH 7.5, and lysed by sonication. Cell lysates are centrifuged at 1000 rpm for 5 minutes at 4 °C. The supernatant is centrifuged at 30,000 x g for 20 minutes at 4 °C. The pellet is suspended in binding buffer containing 50 mM Tris HCl, 5 mM MgSO₄, 1 mM EDTA, 100 mM NaCl, pH 7.5, supplemented with 0.1 % BSA, 2 µg/ml aprotinin, 0.5 mg/ml leupeptin, and 10 µg/ml phosphoramidon. Optimal membrane suspension dilutions, defined as the protein concentration required to bind less than 10 % of the added radioligand, i.e. Alpha-latrotoxin (ALX), are added to 96-well polypropylene microtiter plates containing ¹²⁵I-labeled ligand or test compound, non-labeled peptides, and binding buffer to a final volume of 250 µl.

In equilibrium saturation binding assays, membrane preparations are incubated in the presence of increasing concentrations (0.1 nM to 4 nM) of ¹²⁵I-labeled ligand or test compound (specific activity 2200 Ci/mmol). The binding affinities of different test compounds are determined in equilibrium competition binding assays, using 0.1 nM ¹²⁵I- peptide in the presence of twelve different concentrations of each test compound.

Binding reaction mixtures are incubated for one hour at 30 °C. The reaction is stopped by filtration through GF/B filters treated with 0.5% polyethyleneimine, using a cell harvester. Radioactivity is measured by scintillation counting, and data are analyzed by a computerized non-linear regression program.

Non-specific binding is defined as the amount of radioactivity remaining after incubation of membrane protein in the presence of 100 nM of unlabeled peptide. Protein concentration is measured by the Bradford method using Bio-Rad Reagent, with bovine serum albumin as a standard. A test compound which increases the radioactivity of membrane protein by at least 15% relative to radioactivity of membrane protein which was not incubated with a test compound is identified as a compound which binds to a human LT-GPCR polypeptide.

### EXAMPLE 3

### Effect of a test compound on human LT-GPCR-mediated cyclic AMP formation

Receptor-mediated inhibition of cAMP formation can be assayed in host cells which express human LT-GPCR. Cells are plated in 96-well plates and incubated in Dulbecco's phosphate buffered saline (PBS) supplemented with 10 mM HEPES, 5 mM theophylline, 2 µg/ml aprotinin, 0.5 mg/ml leupeptin, and 10 µg/ml phosphoramidon for 20 minutes at 37 °C in 5% CO₂. A test compound is added and incubated for an additional 10 minutes at 37 °C. The medium is aspirated, and the reaction is stopped by the addition of 100 mM HCl. The plates are stored at 4 °C for 15 minutes. cAMP content in the stopping solution is measured by radioimmunoassay.

Radioactivity is quantified using a gamma counter equipped with data reduction software. A test compound which decreases radioactivity of the contents of a well relative to radioactivity of the contents of a well in the absence of the test compound is identified as a potential inhibitor of cAMP formation. A test compound which increases radioactivity of the contents of a well relative to radioactivity of the contents of a well in the absence of the test compound is identified as a potential enhancer of cAMP formation.

### EXAMPLE 4

### Effect of a test compound on the mobilization of intracellular calcium

Intracellular free calcium concentration can be measured by microspectrofluorometry using the fluorescent indicator dye Fura-2/AM (Bush *et al., J. Neurochem. 57,* 562-74, 1991). Stably transfected cells are seeded onto a 35 mm culture dish containing a glass coverslip insert. Cells are washed with HBS , incubated with a test compound, and loaded with 100 µl of Fura-2/AM (10 µM) for 20-40 minutes. After washing with HBS to remove the Fura-2/AM solution, cells are equilibrated in HBS for 10-20 minutes. Cells are then visualized under the 40X objective of a Leitz Fluovert FS microscope.

Fluorescence emission is determined at 510 nM, with excitation wavelengths alternating between 340 nM and 380 nM. Raw fluorescence data are converted to calcium concentrations using standard calcium concentration curves and software analysis techniques. A test compound which increases the fluorescence by at least 15% relative to fluorescence in the absence of a test compound is identified as a compound which mobilizes intracellular calcium.

### EXAMPLE 5

### Effect of a test compound on phosphoinositide metabolism

Cells which stably express human LT-GPCR cDNA are plated in 96-well plates and grown to confluence. The day before the assay, the growth medium is changed to 100 µl of medium containing 1% serum and 0.5 µCi ³H-myinositol. The plates are incubated overnight in a CO₂ incubator (5% CO₂ at 37 °C). Immediately before the assay, the medium is removed and replaced by 200 µl of PBS containing 10 mM LiCl, and the cells are equilibrated with the new medium for 20 minutes. During this interval, cells also are equilibrated with antagonist, added as a 10 µl aliquot of a 20-fold concentrated solution in PBS.

The ³H-inositol phosphate accumulation from inositol phospholipid metabolism is started by adding 10 µl of a solution containing a test compound. To the first well 10 µl are added to measure basal accumulation. Eleven different concentrations of test compound are assayed in the following 11 wells of each plate row. All assays are performed in duplicate by repeating the same additions in two consecutive plate rows.

The plates are incubated in a CO₂ incubator for one hour. The reaction is terminated by adding 15 µl of 50% v/v trichloroacetic acid (TCA), followed by a 40 minute incubation at 4 °C. After neutralizing TCA with 40 µl of 1 M Tris, the content of the wells is transferred to a Multiscreen HV filter plate (Millipore) containing Dowex AG1-X8 (200-400 mesh, formate form). The filter plates are prepared by adding 200 µl of Dowex AG1-X8 suspension (50% v/v, water:resin) to each well. The filter plates are placed on a vacuum manifold to wash or elute the resin bed. Each well is washed 2 times with 200 µl of water, followed by 2 x 200 µl of 5 mM sodium tetraborate/60 mM ammonium formate.

The ³H-IPs are eluted into empty 96-well plates with 200 µl of 1.2 M ammonium formate/0.1 formic acid. The content of the wells is added to 3 ml of scintillation cocktail, and radioactivity is determined by liquid scintillation counting.

### EXAMPLE 6

### Receptor Binding Methods

Standard Binding Assays. Binding assays are carried out in a binding buffer containing 50 mM HEPES, pH 7.4, 0.5% BSA, and 5 mM MgCl₂. The standard assay for radioligand binding to membrane fragments comprising LT-GPCR polypeptides is carried out as follows in 96 well microtiter plates (e.g., Dynatech Immulon II Removawell plates). Radioligand is diluted in binding buffer+ PMSF/Baci to the desired cpm per 50 µl, then 50 µl aliquots are added to the wells. For non-specific binding samples, 5 µl of 40 µM cold ligand also is added per well. Binding is initiated by adding 150 µl per well of membrane diluted to the desired concentration (10-30 µg membrane protein/well) in binding buffer+ PMSF/Baci. Plates are then covered with Linbro mylar plate sealers (Flow Labs) and placed on a Dynatech Microshaker II. Binding is allowed to proceed at room temperature for 1-2 hours and is stopped by centrifuging the plate for 15 minutes at 2,000 x g. The supernatants are decanted, and the membrane pellets are washed once by addition of 200 µl of ice cold binding buffer, brief shaking, and recentrifugation. The individual wells are placed in 12 x 75 mm tubes and counted in an LKB Gammamaster counter (78% efficiency). Specific binding by this method is identical to that measured when free ligand is removed by rapid (3-5 seconds) filtration and washing on polyethyleneimine-coated glass fiber filters.

Three variations of the standard binding assay are also used.
1. Competitive radioligand binding assays with a concentration range of cold ligand vs. ¹²⁵ I-labeled ligand are carried out as described above with one modification. All dilutions of ligands being assayed are made in 40X PMSF/Baci to a concentration 40X the final concentration in the assay. Samples of peptide (5 µl each) are then added per microtiter well. Membranes and radioligand are diluted in binding buffer without protease inhibitors. Radioligand is added and mixed with cold ligand, and then binding is initiated by addition of membranes.
2. Chemical cross-linking of radioligand with receptor is done after a binding step identical to the standard assay. However, the wash step is done with binding buffer minus BSA to reduce the possibility of non-specific cross-linking of radioligand with BSA. The cross-linking step is carried out as described below.
3. Larger scale binding assays to obtain membrane pellets for studies on solubilization of receptor:ligand complex and for receptor purification are also carried out. These are identical to the standard assays except that (a) binding is carried out in polypropylene tubes in volumes from 1-250 ml, (b) concentration of membrane protein is always 0.5 mg/ml, and (c) for receptor purification, BSA concentration in the binding buffer is reduced to 0.25%, and the wash step is done with binding buffer without BSA, which reduces BSA contamination of the purified receptor.

### EXAMPLE 7

### Chemical Cross-Linking of Radioligand to Receptor

After a radioligand binding step as described above, membrane pellets are resuspended in 200 µl per microtiter plate well of ice-cold binding buffer without BSA. Then 5 µl per well of 4 mM N-5-azido-2-nitrobenzoyloxysuccinimide (ANB-NOS, Pierce) in DMSO is added and mixed. The samples are held on ice and UV-irradiated for 10 minutes with a Mineralight R-52G lamp (UVP Inc., San Gabriel, Calif.) at a distance of 5-10 cm. Then the samples are transferred to Eppendorf microfuge tubes, the membranes pelleted by centrifugation, supernatants removed, and membranes solubilized in Laemmli SDS sample buffer for polyacrylamide gel electrophoresis (PAGE). PAGE is carried out as described below. Radiolabeled proteins are visualized by autoradiography of the dried gels with Kodak XAR film and Dupont image intensifier screens.

### EXAMPLE 8

### Membrane Solubilization

Membrane solubilization is carried out in buffer containing 25 mM Tris , pH 8, 10% glycerol (w/v) and 0.2 mM CaCl₂ (solubilization buffer). The highly soluble detergents including Triton X-100, deoxycholate, deoxycholate:lysolecithin, CHAPS, and zwittergent are made up in solubilization buffer at 10% concentrations and stored as frozen aliquots. Lysolecithin is made up fresh because of insolubility upon freeze-thawing and digitonin is made fresh at lower concentrations due to its more limited solubility.

To solubilize membranes, washed pellets after the binding step are resuspended free of visible particles by pipetting and vortexing in solubilization buffer at 100,000 x g for 30 minutes. The supernatants are removed and held on ice and the pellets are discarded.

### EXAMPLE 9

### Assay of Solubilized Receptors

After binding of ¹²⁵I ligands and solubilization of the membranes with detergent, the intact R:L complex can be assayed by four different methods. All are carried out on ice or in a cold room at 4-10 °C.
1. Column chromatography (Knuhtsen *et al., Biochem. J. 254,* 641-647, 1988). Sephadex G-50 columns (8 x 250 mm) are equilibrated with solubilization buffer containing detergent at the concentration used to solubilize membranes and 1 mg/ml bovine serum albumin. Samples of solubilized membranes (0.2-0.5 ml) are applied to the columns and eluted at a flow rate of about 0.7 ml/minute. Samples (0.18 ml) are collected. Radioactivity is determined in a gamma counter. Void volumes of the columns are determined by the elution volume of blue dextran. Radioactivity eluting in the void volume is considered bound to protein. Radioactivity eluting later, at the same volume as free ¹²⁵I ligands, is considered non-bound.
2. Polyethyleneglycol precipitation (Cuatrecasas, *Proc. Natl. Acad. Sci. USA 69,* 318-322, 1972). For a 100 µl sample of solubilized membranes in a 12 x 75 mm polypropylene tube, 0.5 ml of 1% (w/v) bovine gamma globulin (Sigma) in 0.1 M sodiumphosphate buffer is added, followed by 0.5 ml of 25% (w/v) polyethyleneglycol (Sigma) and mixing. The mixture is held on ice for 15 minutes. Then 3 ml of 0.1 M sodium phosphate, pH 7.4, is added per sample. The samples are rapidly (1-3 seconds) filtered over Whatman GF/B glass fiber filters and washed with 4 ml of the phosphate buffer. PEG-precipitated receptor : ¹²⁵ I-ligand complex is determined by gamma counting of the filters.
3. GFB/PEI filter binding (Bruns *et al., Analytical Biochem. 132,* 74-81, 1983). Whatman GF/B glass fiber filters are soaked in 0.3% polyethyleneimine (PEI, Sigma) for 3 hours. Samples of solubilized membranes (25-100 µl) are replaced in 12 x 75 mm polypropylene tubes. Then 4 ml of solubilization buffer without detergent is added per sample and the samples are immediately filtered through the GFB/PEI filters (1-3 seconds) and washed with 4 ml of solubilization buffer. CPM of receptor : ¹²⁵ I-ligand complex adsorbed to filters are determined by gamma counting.
4. Charcoal/Dextran (Paul and Said, *Peptides 7[Suppl. 1]*,147-149, 1986). Dextran T70 (0.5 g, Pharmacia) is dissolved in 1 liter of water, then 5 g of activated charcoal (Norit A, alkaline; Fisher Scientific) is added. The suspension is stirred for 10 minutes at room temperature and then stored at 4 °C. until use. To measure R:L complex, 4 parts by volume of charcoal/dextran suspension are added to 1 part by volume of solubilized membrane. The samples are mixed and held on ice for 2 minutes and then centrifuged for 2 minutes at 11,000 x g in a Beckman microfuge. Free radioligand is adsorbed charcoal/dextran and is discarded with the pellet. Receptor : ¹²⁵ I-ligand complexes remain in the supernatant and are determined by gamma counting.

### EXAMPLE 10

### Receptor Purification

Binding of biotinyl-receptor to GH₄ Cl membranes is carried out as described above. Incubations are for 1 hour at room temperature. In the standard purification protocol, the binding incubations contain 10 nM Bio-S29. ¹²⁵I ligand is added as a tracer at levels of 5,000-100,000 cpm per mg of membrane protein. Control incubations contain 10 µM cold ligand to saturate the receptor with non-biotinylated ligand.

Solubilization of receptor:ligand complex also is carried out as described above, with 0.15% deoxycholate:lysolecithin in solubilization buffer containing 0.2 mM MgCl₂, to obtain 100,000 x g supernatants containing solubilized R:L complex.

Immobilized streptavidin (streptavidin cross-linked to 6% beaded agarose, Pierce Chemical Co.; "SA-agarose") is washed in solubilization buffer and added to the solubilized membranes as 1/30 of the final volume. This mixture is incubated with constant stirring by end-over-end rotation for 4-5 hours at 4-10 °C. Then the mixture is applied to a column and the non-bound material is washed through. Binding of radioligand to SA-agarose is determined by comparing cpm in the 100,000 x g supernatant with that in the column effluent after adsorption to SA-agarose. Finally, the column is washed with 12-15 column volumes of solubilization buffer+0.15% deoxycholate:lysolecithin +1/500 (vol/vol) 100 x 4pase.

The streptavidin column is eluted with solubilization buffer+0.1 mM EDTA+0.1 mM EGTA+0.1 mM GTP-gamma-S (Sigma)+0.15% (wt/vol) deoxycholate:lysolecithin +1/1000 (vol/vol) 100.times.4pase. First, one column volume of elution buffer is passed through the column and flow is stopped for 20-30 minutes. Then 3-4 more column volumes of elution buffer are passed through. All the eluates are pooled.

Eluates from the streptavidin column are incubated overnight (12-15 hours) with immobilized wheat germ agglutinin (WGA agarose, Vector Labs) to adsorb the receptor via interaction of covalently bound carbohydrate with the WGA lectin. The ratio (vol/vol) of WGA-agarose to streptavidin column eluate is generally 1:400. A range from 1:1000 to 1:200 also can be used. After the binding step, the resin is pelleted by centrifugation, the supernatant is removed and saved, and the resin is washed 3 times (about 2 minutes each) in buffer containing 50 mM HEPES, pH 8, 5 mM MgCl₂, and 0.15% deoxycholate:lysolecithin. To elute the WGA-bound receptor, the resin is extracted three times by repeated mixing (vortex mixer on low speed) over a 15-30 minute period on ice, with 3 resin columns each time, of 10 mM N-N'-N"-triacetylchitotriose in the same HEPES buffer used to wash the resin. After each elution step, the resin is centrifuged down and the supernatant is carefully removed, free of WGA-agarose pellets. The three, pooled eluates contain the final, purified receptor. The material non-bound to WGA contain G protein subunits specifically eluted from the streptavidin column, as well as non-specific contaminants. All these fractions are stored frozen at -90 °C.

### EXAMPLE 11

### Identification of test compounds that bind to LT-GPCR polypeptides

Purified LT-GPCR polypeptides comprising a glutathione-S-transferase protein and absorbed onto glutathione-derivatized wells of 96-well microtiter plates are contacted with test compounds from a small molecule library at pH 7.0 in a physiological buffer solution. LT-GPCR polypeptides comprise an amino acid sequence shown in SEQ ID NO:2. The test compounds comprise a fluorescent tag. The samples are incubated for 5 minutes to one hour. Control samples are incubated in the absence of a test compound.

The buffer solution containing the test compounds is washed from the wells. Binding of a test compound to an LT-GPCR polypeptide is detected by fluorescence measurements of the contents of the wells. A test compound which increases the fluorescence in a well by at least 15% relative to fluorescence of a well in which a test compound was not incubated is identified as a compound which binds to an LT-GPCR polypeptide.

### EXAMPLE 12

### Identification of a test compound which decreases LT-GPCR gene expression

A test compound is administered to a culture of human gastric cells and incubated at 37 °C for 10 to 45 minutes. A culture of the same type of cells incubated for the same time without the test compound provides a negative control.

RNA is isolated from the two cultures as described in Chirgwin *et al*., *Biochem. 18,* 5294-99, 1979). Northern blots are prepared using 20 to 30 µg total RNA and hybridized with a ³²P-labeled LT-GPCR -specific probe at 65 ° C in Express-hyb (CLONTECH). The probe comprises at least 11 contiguous nucleotides selected from the complement of SEQ ID NO:1. A test compound which decreases the LT-GPCR-specific signal relative to the signal obtained in the absence of the test compound is identified as an inhibitor of LT-GPCR gene expression.

### EXAMPLE 13

### Treatment of Zollinger-Ellison syndrome with a reagent which specifically binds to an LT- GPCR gene product

Synthesis of antisense LT-GPCR oligonucleotides comprising at least 11 contiguous nucleotides selected from the complement of SEQ ID NO:1 is performed on a Pharmacia Gene Assembler series synthesizer using the phosphoramidite procedure (Uhlmann *et al., Chem. Rev. 90,* 534-83, 1990). Following assembly and deprotection, oligonucleotides are ethanol-precipitated twice, dried, and suspended in phosphate-buffered saline (PBS) at the desired concentration. Purity of these oligonucleotides is tested by capillary gel electrophoreses and ion exchange HPLC. Endotoxin levels in the oligonucleotide preparation are determined using the *Limulus* Amebocyte Assay (Bang, *Biol. Bull. (Woods Hole, Mass.) 105*, 361-362, 1953).

The antisense oligonucleotides are administered to a patient with Zollinger-Ellison syndrome. The severity of the patient's Zollinger-Ellison syndrome is lessened.

## Claims

1. A method of screening for agents which decrease the activity of a LT-GPCR, comprising the steps of
i) contacting a test compound with the LT-GPCR polypeptide encoded by any polynucleotide being selected from the group consisting of:
a) a polynucleotide encoding a LT-GPCR polypeptide comprising the amino acid sequence shown in SEQ ID NO: 2; and
b) a polynucleotide comprising the sequence of SEQ ID NO: 1;
c) a polynucleotide the sequence of which deviates from the polynucleotide sequences specified in (a) to (b) due to the degeneration of the genetic code; and
ii) detecting binding of the test compound of the LT-GPCR polypeptide, wherein a test compound which binds to the polypeptide is identified as a potential therapeutic agent for decreasing the activity of a LT-GPCR.

2. A method of screening for agents which regulate the activity of a LT-GPCR, comprising the steps of:
i) contacting a test compound with a LT-GPCR polypeptide encoded by any polynucleotide specified in claim 1; and
ii) detecting a LT-GPCR activity of the polypeptide, wherein a test compound which increases the LT-GPCR activity is identified as a potential therapeutic agent for increasing the activity of the LT-GPCR, and wherein a test compound which decreases the LT-GPCR activity of the polypeptide is identified as a potential therapeutic agent for decreasing the activity of the LT-GPCR.

3. A method of screening for agents which decrease the activity of a LT-GPCR, comprising the steps of:
i) contacting a test compound with any polynucleotide specified in claim 1 and
ii) detecting binding of the test compound to the polynucleotide, wherein a test compound which binds to the polynucleotide is identified as a potential therapeutic agent for decreasing the activity of LT-GPCR.

## Patentansprüche

1. Verfahren zum Screenen auf Mittel, die die Aktivität eines LT-GPCR senken, die folgenden Schritte umfassend:
i) das Kontaktieren einer Testverbindung mit dem LT-GPCR-Polypeptid, für das jedes beliebige Polynucleotid kodiert, das aus der aus:
a) einem Polynucleotid, das für ein LT-GPCR-Polypeptid kodiert, das die in Seq.-ID Nr. 2 gezeigte Aminosäuresequenz umfasst;
b) einem Polynucleotid, das die Sequenz aus Seq.-ID Nr. 1 umfasst; und
c) einem Polynucleotid, dessen Sequenz von den in (a) und (b) beschriebenen Polynucleotidsequenzen aufgrund der Degeneration des genetischen Codes abweicht;
bestehenden Gruppe ausgewählt ist; und
ii) das Detektieren von Bindung der Testverbindung des LT-GPCR-Polypeptids, worin eine Testverbindung, die sich an das Polypeptid bindet, als ein potenzielles therapeutisches Mittel zur Senkung der Aktivität eines LT-GPCR identifiziert wird.

2. Verfahren zum Screenen auf Mittel, die die Aktivität eines LT-GPCR regulieren, die folgenden Schritte umfassend:
i) das Kontaktieren einer Testverbindung mit einem LT-GPCR-Polypeptid, für das jedes beliebige, in Anspruch 1 definierte Polynucleotid kodiert; und
ii) das Detektieren einer LT-GPCR-Aktivität des Polypeptids, worin eine Testverbindung, die die LT-GPCR-Aktivität steigert, als ein potenzielles therapeutisches Mittel zur Steigerung der Aktivität von LT-GPCR identifiziert wird und worin eine Testverbindung, die die LT-GPCR-Aktivität des Polypeptids senkt, als ein potenzielles therapeutisches Mittel zur Senkung der Aktivität des LT-GPCR identifiziert wird.

3. Verfahren zum Screenen auf Mittel, die die Aktivität eines LT-GPCR senken, die folgenden Schritte umfassend:
i) das Kontaktieren einer Testverbindung mit einem beliebigen, in Anspruch 1 definierten Polynucleotid; und
ii) das Detektieren von Bindung der Testverbindung an das Polynucleotid, worin eine Testverbindung, die sich an das Polynucleotid bindet, als ein potenzielles therapeutisches Mittel zur Senkung der Aktivität eines LT-GPCR identifiziert wird.

## Revendications

1. Méthode pour sélectionner des agents qui diminuent l'activité d'un LT-GPCR, comprenant les étapes :
i) de mise en contact d'un composé d'essai avec le polypeptide LT-GPCR codé par n'importe quel polynucléotide choisi dans le groupe consistant en :
a) un polynucléotide codant pour un polypeptide LT-GPCR comprenant la séquence d'aminoacides représentée dans la SEQ ID N° 2 ; et
b) un polynucléotide comprenant la séquence de la SEQ ID N° 1 ;
c) un polynucléotide dont la séquence s'écarte des séquences de polynucléotides spécifiées en (a) et (b) en raison de la dégénérescence du code génétique ; et
ii) de détection de la liaison du composé d'essai avec le polypeptide LT-GPCR, un composé d'essai qui se lie au polypeptide étant identifié en tant qu'agent thérapeutique potentiel pour diminuer l'activité d'un LT-GPCR.

2. Méthode pour sélectionner des agents qui régulent l'activité d'un LT-GPCR, comprenant les étapes :
i) de mise en contact d'un composé d'essai avec un polypeptide LT-GPCR codé par n'importe quel polynucléotide spécifié dans la revendication 1 ; et
ii) de détection d'une activité de LT-GPCR du polypeptide, un composé d'essai qui augmente l'activité de LT-GPCR étant identifié en tant qu'agent thérapeutique potentiel pour augmenter l'activité du LT-GPCR, et un composé d'essai qui diminue l'activité de LT-GPCR du polypeptide étant identifié en tant qu'agent thérapeutique potentiel pour diminuer l'activité du LT-GPCR.

3. Méthode pour sélectionner des agents qui diminuent l'activité d'un LT-GPCR, comprenant les étapes :
i) de mise en contact d'un composé d'essai avec n'importe quel polynucléotide spécifié dans la revendication 1 ; et
ii) de détection de la liaison du composé d'essai au polynucléotide, un composé d'essai qui se lie au polynucléotide étant identifié en tant qu'agent thérapeutique potentiel pour diminuer l'activité du LT-GPCR.
